(19) Europäisches Patentamt / European Patent Office / Office européen des brevets

(11) **EP 3 353 697 B1**

(12) **EUROPEAN PATENT SPECIFICATION**

(45) Date of publication and mention of the grant of the patent:
**05.11.2025 Bulletin 2025/45**

(21) Application number: **16849025.8**

(22) Date of filing: **23.09.2016**

(51) International Patent Classification (IPC):
$C12Q\ 1/682^{(2018.01)}$ $\quad C12Q\ 1/6844^{(2018.01)}$
$G16B\ 30/00^{(2019.01)}$ $\quad G16B\ 40/00^{(2019.01)}$

(52) Cooperative Patent Classification (CPC):
(C-Sets available)
**G16B 25/20; C12Q 1/6844; G16B 30/00;**
**G16B 40/00; G16B 40/10** (Cont.)

(86) International application number:
**PCT/KR2016/010708**

(87) International publication number:
**WO 2017/052300 (30.03.2017 Gazette 2017/13)**

(54) **MULTIPLE DATASET ANALYSIS FOR DETERMINING THE PRESENCE OR ABSENCE OF TARGET ANALYTE**

ANALYSE MEHRERER DATENSÄTZE ZUR BESTIMMUNG DES VORHANDENSEINS ODER NICHTVORHANDENSEINS VON ZIELANALYTEN

ANALYSE DE MULTIPLES ENSEMBLES DE DONNÉES POUR DÉTERMINER LA PRÉSENCE OU L'ABSENCE D'ANALYTE CIBLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.09.2015 KR 20150135386**

(43) Date of publication of application:
**01.08.2018 Bulletin 2018/31**

(73) Proprietor: **Seegene, Inc.**
**Seoul 05548 (KR)**

(72) Inventors:
• **CHUN, Jong Yoon**
**Seoul 06075 (KR)**
• **LEE, Young Jo**
**Seoul 05507 (KR)**

(74) Representative: **Engelhard, Markus**
**Boehmert & Boehmert**
**Anwaltspartnerschaft mbB**
**Pettenkoferstrasse 22**
**80336 München (DE)**

(56) References cited:
| | |
|---|---|
| EP-A- 1 798 650 | EP-A- 2 653 558 |
| US-A- 2005 130 211 | US-A- 2013 280 712 |
| US-A- 2015 087 531 | US-A- 2015 186 598 |
| US-B2- 8 560 247 | |

(52) Cooperative Patent Classification (CPC): (Cont.)

C-Sets
C12Q 1/6844, C12Q 2537/165

**Description**

**BACKGROUND OF THE INVENTION**

**FIELD OF THE INVENTION**

The present invention relates to amultiple dataset analysis (MDA) for determining the presence or absence of a target analyte.

**DESCRIPTION OF THE RELATED ART**

Nucleic acid amplification is a pivotal process for a wide variety of methods in molecular biology, such that various amplification methods have been proposed. For example, Miller, H. I. et al. (WO 89/06700) amplified a nucleic acid sequence based on the hybridization of a promoter/primer sequence to a target single-stranded DNA ("ssDNA") followed by transcription of many RNA copies of the sequence. Other known nucleic acid amplification procedures include transcription-based amplification systems (Kwoh, D. et al., Proc. Natl. Acad. Sci. U.S.A., 86:1173(1989); and Gingeras T.R. et al., WO 88/10315).

The most predominant process for a nucleic acid amplification known as polymerase chain reaction (hereinafter referred to as "PCR") is based on repeated cycles of denaturation of double-stranded DNA, followed by oligonucleotide primer annealing to the DNA template, and primer extension by a DNA polymerase (Mullis et al. U.S. Pat. Nos. 4,683,195, 4,683,202, and 4,800,159; Saiki et al., (1985) Science 230, 1350-1354).

Among these methods, a real-time PCR technique is widely used for detecting the amplification of a target nucleic acid sequence in a real time manner. For detecting a target nucleic acid sequence of interest, the real-time PCR employs a signal-generating means for releasing a detectable fluorescent signal in proportion to the amount of target nucleic acid sequences. The release of the detectable fluorescent signal may be accomplishedby using either anintercalator which generatesa fluorescent signalwhen bound to double-strandedDNA, or by using an oligonucleotide carrying a fluorescent reporter molecule and a quencher molecule capable of inhibiting the fluorescence of said fluorescent reporter molecule. It measures fluorescent signalswhose intensities are proportional to the amount of the target nucleic acid sequence at each cycle, thereby generating a raw dataset having a pair of coordinate values of a cycle number and a signal intensity. For convenience, the intensity values may be then plotted *vs.* cycle numbers to obtain an amplification curve (also referred to "amplification profile curve"), which is well known in the art.

An amplification curve of the real-time PCRtypically consists of three regions: baseline region, exponential region, and plateau region. The exponential region refers to a region where released fluorescent signals increase in proportional to increase in amplification products (amplicons). The plateau region refers to a region where there is little increase in fluorescent signals due to saturation of both PCR amplicons and fluorescent signal levels. The baseline region refers to a region where there is little change in fluorescent signal during initial cycles of PCR.Since the fluorescent signals from PCR amplicons are not sufficient to be detectable in the baseline region, signals detected in this region may be mainly due to signals (background signal) from a reaction sample and a detection system, not signals from the amplification.

For analyzing data of the real-time PCR in more accurate and reproducible manner, data processing is required, including smoothing (or fitting, approximation, and the like), baseline subtraction (baselining), and/or normalization (*see*Alexey Larionovet al., BMC Bioinformatics 2005, 6:62; U.S Patent No.7,720,611). EP 2 653 558 discloses a method for detecting the presence or absence of a target nucleic acid (TNA) which uses only one type of amplification data (i.e raw dataset) for determining the presence/absence of a TNA.

EP 1 798 650 discloses a method for detecting the presence or absence of a target nucleic acid (TNA) which uses mathematically processed amplification data for determining the presence/absence of a TNA.

Conventional methods for determiningthe presence or absence of target analytecomprises applying a predefined signal threshold to an amplification curve, and identifying a cycle having a signal value that exceeds such signal threshold. In such methods, when there is a signal value that exceedsthe threshold, it indicates the amplification of the target nucleic acid sequence and thus the presence of a target nucleic acid sequence can be determined, and otherwise the absence target nucleic acid sequence can be determined.

A signal threshold used to determine the presence or absence of a target nucleic acid sequence can be set as an intensity of fluorescent signal at any cycleduring amplification proceeds, *e.g.*, anend-point cycle of the baseline region, a start-pointcycle of theexponential region, or any cycle within the exponential region. It is involved in the sensitivity of real-time PCR. Ifthesignal threshold isset too high, a sample containing an initial low concentration of target nucleic acid sequences (positive sample) or a sample with poor amplification efficiency may be mistakenly decided to be a negative sample (false negative error). In contrast, if a signal threshold is set too low, a sample containing no target nucleic acid sequence (negative sample) may be mistakenly decided to be a positive sample, due to noises (false positive error). Therefore, there is a need to set an optimal signal threshold in order to minimize such false negative and false positive errors.

Despite these efforts, it is impossible to fundamentally eliminate all errors occurred during sample preparation, PCR reaction, and data processing. Thus, there remain long-felt needs in the art to develop novel approaches for detection of a target nucleic acid sequence in a more accurate manner.

Recently, several technologies have been developed for analyzing data in a more accurate manner by eliminating errors from a real-time PCR dataset. For example, U.S. Patent No. 8,560,247 discloses a method for determining non-amplification data, *i.e.,* errors (or noise, jump, etc.) where a slope of a particular amplification data exceeds a maximum amplification slope. Further, U.S. Patent Application No. 2015/0186598 discloses a method for detecting jump errors in an amplification curve by identifying two consecutive cycle numbers having values of a second derivative that have different signs.

Although the prior arts disclose methods for the removal or correction of some data decided as error data, such correction is likely to cause conversion of normal data into rather abnormal data as well as data distortion. In addition, such methods cannot identify all possible errors, which are not effective in the correct data analysis.

## SUMMARY OF THE INVENTION

The present inventors have made intensive researches to minimize errors (particularly, false positive errors) occurring frequently in determining the presence or absence of a target analyte in a sample. As a result, we have established novel protocols allowing for error-free amplification analysis, which comprises performing an amplification reaction using a signal-generating means for a target analyte, providing (i) a dataset pool comprising different types of datasets representing an outcome of the amplification reaction and (ii) two or more determinative factors for determining the presence or absence of the target analyte, and determining the presence or absence of the target analyte in the sample by using at least two of the determinative factors.

Accordingly, it is an object of this invention as defined by the appended claims to provide a method for determining the presence or absence of a target analyte in a sample.

It is other object of this invention to provide a computer readable storage medium containing instructions to configure a processor to perform a method for determining the presence or absence of a target analyte in a sample.

It is another object of this invention to provide a device for determining the presence or absence of a target analyte in a sample.

It is still another object of this invention to provide a computer program to be stored in a computer readable storage medium to configure a processor to perform a method for determining the presence or absence of a target analyte in a sample.

Other objects and advantages of the present invention will become apparent from the detailed description to follow, taken in conjunction with the appended claims and drawings.

## BRIEF DESCRIPTION OF THE DRAWINGS

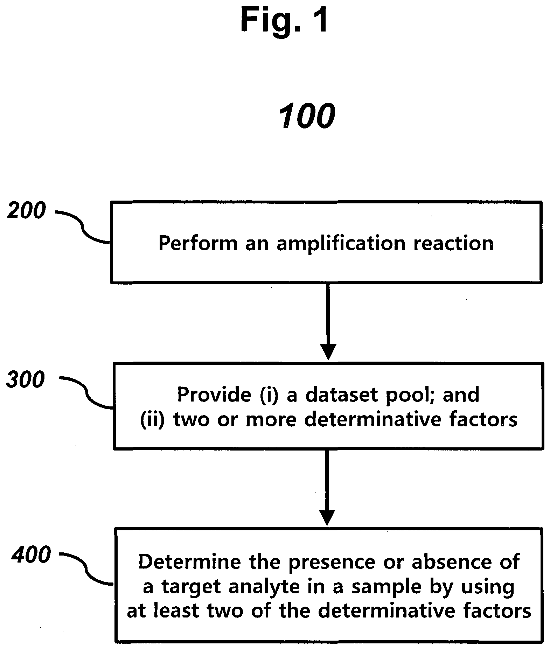

Fig. 1 is a flow chart showing a representative of a multiple dataset analysis (MDA) according to the present invention. as defined by the appended claims

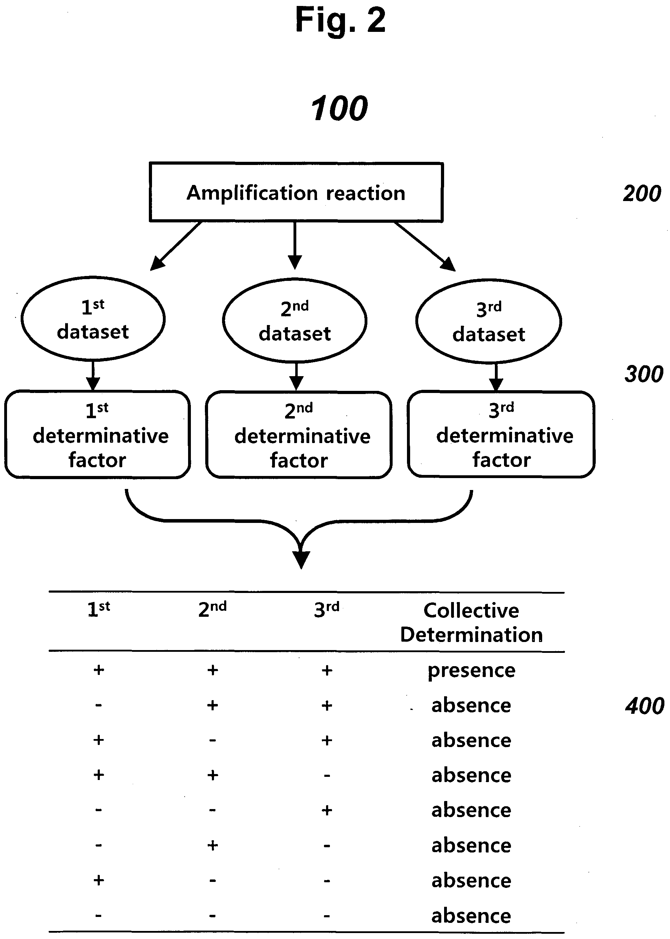

Fig. 2 is a flow chart showing an embodiment of the MDA based on a collective determination approach. Three datasets are obtained from an amplification reaction (200), and three determinative factors are obtained for the datasets (300). Afterwards, the three determinative factors are collectively assessed to determine the presence or absence of a target analyte in a sample.

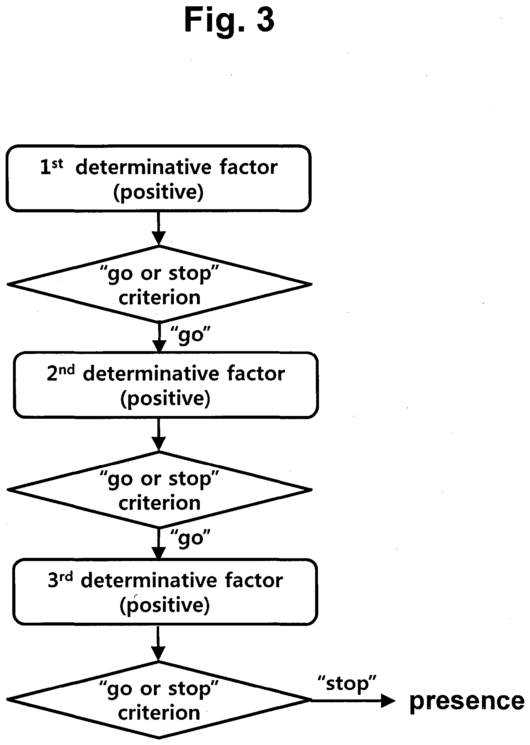

Fig. 3 is a flow chart showing an embodiment of the MDA based on a sequential determination approach. According to the sequential determination approach, each determinative factor is sequentially assessed whether it satisfies a "go or stop" criterion. The "go or stop" criterion comprises (i) a "stop" criterion defined by which a determinative factor is negative, (ii) a "stop" criterion defined by which a determinative factor is positive and a next determinative factor is no longer present, and (iii) a "go" criterion defined by which a determinative factor is positive and a next determinative factor is present; wherein when the determinative factor at a certain order satisfies the "stop" criterion defined by (i), the absence of the target analyte is determined; when it satisfies the "stop" criterion defined by (ii), the presence of the target analyte is determined; and when it satisfies the "go" criterion defined by (iii), a next determinative factor is then assessed. In Fig. 3, a first determinative factor is assessed whether it satisfies the "go or stop" criterion. As the first determinative factor is positive ("+") and a next determinative factor (*e.g.,* 2nd determinative factor) is present, it satisfies the "go" criterion defined by (iii). Therefore, a next second determinative factor is assessed whether it satisfies the "go or stop" criterion. As the next second determinative factor is positive ("+") and a next determinative factor (*e.g.,* 3rd determinative factors) is present, it satisfies the "go" criterion defined by (iii). Therefore, a next third determinative factor is assessed whether it satisfies the "go or stop" criterion. As the next third determinative factor is positive ("+") and a next determinative factor is not present, it satisfies the "stop" criterion defined by (ii). Therefore, the presence of a target analyte in a sample may be determined.

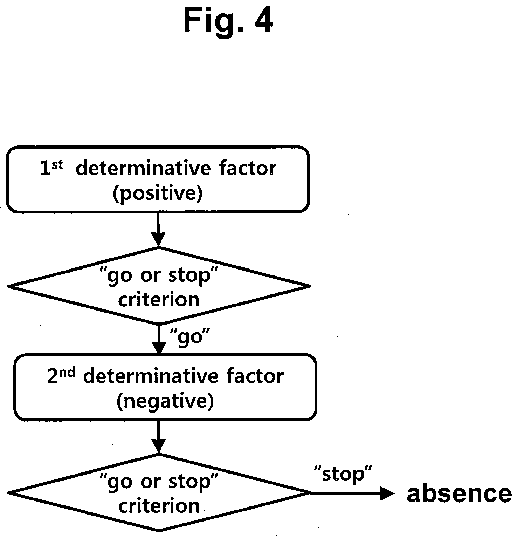

Fig. 4 is a flow chart showing another embodiment of the MDA based on a sequential determination approach. In Fig. 4, a first determinative factor is assessed whether it satisfies the "go or stop" criterion. As the first determinative factor is positive ("+") and a next determinative factor (e.g., 2nd determinative factor) is present, it satisfies the "go" criterion defined by (iii). Therefore, a next second determinative factor is assessed whether it satisfies the "go or stop" criterion. As the next second determinative factor is negative ("-"), it satisfies the "stop" criterion defined by (i). Therefore, the absence of a target analyte in a sample may be determined without assessing a next determinative factor (e.g., 3rd determinative factor).

## DETAILED DESCRIPTION OF THIS INVENTION

In the Specification, descriptions for common technologies and knowledge well known in the art and directly unrelated to the present invention are omitted such that the Specification becomes more descriptive and explanatory for the present invention. defined by the appended claims. Furthermore, the common descriptions between the Sections described below are omitted in order to avoid undue redundancy leading to the complexity of this Specification.

### I. **Method for determination of the presence or absence of a target analyte**

The present invention is defined by the appended claims

The present inventors have made intensive researches to minimize errors (particularly, false positive errors) occurring frequently in determining the presence or absence of a target analyte in a sample. As a result, we have established novel protocols allowing for error-free amplification analysis, which comprises performing an amplification reaction using a signal-generating means for a targetanalyte, providing (i) a dataset pool comprising different types of datasets representing an outcome of the amplification reaction and (ii) two or more determinative factors for determining the presence or absence of the target analyte, and determining the presence or absence of the target analyte in the sample by using at least two of the determinative factors.

The most prominent feature of the present invention is to determine the presence or absence of a target analyte by using two or more different types of datasets. Accordingly, the method of the present invention is also referred to "multiple dataset analysis (MDA)".

The present invention defined by the appended claims relates to a method for determining the presence or absence of a target analyte in a sample. The term "determination of the presence or absence of a target analyte in a sample" as used herein refers to qualitative determination of the presence of the target analyte in a sample. Particularly, the determination of the presence or absence of a target analyte in a sample also refers to performing an amplification reaction, e.g., a polymerase chain reaction (PCR) or a real-time PCR, for a target analyte of interest, followed by determining the presence or absence of the target analyte by evaluating (analyzing) datasets derived from the amplification reaction.

The method for determining the presence or absence of a target analyte in a sample by an MDA in accordance with the present invention will be described in detail, with reference to the appended drawings. The MDA (100) comprises three steps as follows:

### Step (a): Performing amplification Reaction (200)

In the present step, an amplification reaction is performed using a signal-generating means for a target analyte (see Fig. 1; 200).

The term "target analyte" as used herein refers to any material to be detected or analyzed, includinga variety of materials (e.g., biological materials and non-biological materials), particularly biological materials, more particularly nucleic acid molecules (e.g., DNA and RNA), proteins, peptides, carbohydrates, lipids, amino acids, biological chemicals, hormones, antibodies, antigens, metabolites, and cells. Most particularly, the target analyte is a target nucleic acid molecule (or target nucleic acid sequence). The target analyte is present in a sample.

The tem "sample" as used herein includes biological samples (e.g., cells, tissues and body fluids) and non-biological samples (e.g., food, water, and soil). The biological samples include, without limitation, virus, bacteria, tissue, cell, blood (including whole blood, plasma and serum), lymph, bone marrow fluid, saliva, sputum, swab, aspiration, milk, urine, feces, ocular fluid, semen, brain extracts, spinal cord fluid (SCF), joint fluid, thymic fluid, bronchoalveolar lavage fluid,amniotic fluid, and asciticfluid. The sample may be subjected to a nucleic acid extraction for an efficient amplification reaction, as well known in the art (seeSambrook, J. et al., Molecular Cloning. A Laboratory Manual, 3rd ed. Cold Spring Harbor Press(2001)).The procedure of nucleic acid extraction may depend on the types of samples. In addition, if the extracted nucleic acid is RNA, it may be further subjected to a reverse transcription for synthesis of cDNA (seeSambrook,

J. et al., Molecular Cloning. A Laboratory Manual, 3rd ed. Cold Spring Harbor Press(2001)).

The term "signal-generating means" as used herein refers to any means (*e.g.,* material) used in the generation of signals indicating the presence of the target analyte.

A wide variety of signal generating means are well known in the art. The signal generating means includes labels*per se,* or oligonucleotides with labels. The labelsincludea fluorescent label, a luminescent label, a chemiluminescent label, anelectrochemical label and a metal label. The label *per se* such as an intercalating dye may serve as signal-generating means. Alternatively, a single label or an interactive dual label containing a donor molecule and an acceptor molecule may be used as signal-generating means in the form of linkage to at least one oligonucleotide. The signal-generating means may further comprise nucleolyticenzymes (*e.g.,* 5' nuclease or 3' nuclease) for generating signals.

A number of methods for generating signals indicating the presence of a target analyte, particularly target nucleic acid molecule, are known in the art. A representative example includes: TaqMan™probe method (U.S. Patent No. 5,210,015), Molecular beacon method (Tyagiet al., Nature Biotechnology v.14 MARCH 1996), Scorpion method (Whitcombe et al., Nature Biotechnology 17:804-807(1999)), Sunrise or Amplifluor method (Nazarenkoet al., 2516-2521 Nucleic Acids Research, 25(12):2516-2521(1997), andU.S. Patent No. 6,117,635), Lux method (U.S. Patent No. 7,537,886), CPT(Duck P, et al.,Biotechniques, 9:142-148(1990)), LNA method (U.S. Patent No. 6,977,295), Plexormethod (Sherrill CB, et al., Journal of the American Chemical Society, 126:4550-4556(2004)), Hybeacons™(D. J. French, et al., Molecular and Cellular Probes (2001) 13, 363-374 andU.S. Patent No. 7,348,141), dual-labeled, self-quenched probe (U.S. Patent No. 5,876,930), Hybridization probe (Bernard PS, et al., ClinChem 2000, 46, 147-148), PTOCE(PTO cleavage and extension) method (WO 2012/096523), PCE-SH(PTO Cleavage and Extension-Dependent Signaling Oligonucleotide Hybridization) method (WO 2013/115442), PCE-NH(PTO Cleavage and Extension-Dependent Non-Hybridization) method (WO 2014/104818) and CER method (WO 2011/037306). Therefore, the amplification reaction in the present invention may be carried out by one of the signal generating methods as described above.

The term "amplification reaction" herein refers to a reaction of increasing or decreasing a signal generated by the signal-generating means.

According to an embodiment of the present invention, the amplification reaction refers to increase in signals (or amplification) by the signal-generating means, in a dependent manner on the presence of the target analyte. Such amplification reaction may be accompanied with or without amplification of the target analyte (*e.g.,* nucleic acid molecule). More particularly, the amplification reaction of the present invention refers to amplification of signals accompanied with amplification of target analyte.

According to an embodiment of the present invention, the amplification reaction to amplify signals indicative of the presence of the target analyte (*e.g.,* nucleic acid molecule) may be performed in such a manner that signals are amplified simultaneously with amplification of target analyte (*e.g.,* real-time PCR). Alternatively, the amplification reaction may be performed in such a manner that signals are amplified without amplification of target analyte(*e.g.,* CPT method (Duck P, et al., Biotechniques, 9:142-148 (1990)), Invader assay (U.S. Pat. Nos. 6,358,691 and 6,194,149)).

A variety of methods for amplifying a target nucleic acid sequence are well known in the art, including polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)) and Q-Beta Replicase (Lizardi et al., BiolTechnology 6:1197 (1988)).

According to an embodiment of the present invention, the signal-generating means comprises a fluorescent label, more particularly a fluorescentsingle label or a fluorescent dual label (*e.g.,* containing fluorescent reporter molecule and quencher molecule). According to an embodiment of the present invention, the amplification reaction amplifies signalssimultaneously with amplification of the target analyte (*e.g.,* target nucleic acid molecule). According to an embodiment of the present invention, the amplification reaction is carried out by a PCR, specifically a real-time PCR.

Since the present method is characterized by analyzing the target analyte using multiple datasets obtained from the amplification reaction, the method is not limited by reaction elements such as a type of samples, a type of signal-generating means, reaction conditions and apparatus. The present method may be applied to any amplification reactions so long as the amplification reactions provide datasets capable of determining the presence or absence of target analytes.

### Step (b): Providing a dataset pool and determinative factors (300)

In the present step, (i) a dataset pool comprising different types of datasets representing an outcome of the amplification reaction and (ii) two or more determinative factors for determining the presence or absence of the target analyte are provided.Thedeterminative factors are obtained by evaluating with predefined criteria two or more datasets selected from the dataset pool; wherein each determinative factor is obtained from each type of dataset; wherein each of the criteria is predefined for each type of the datasets.

(i) Provision of a dataset pool

First, provided in the step isa dataset pool comprising different types of datasets representing an outcome of the amplification reaction.

The term "dataset pool" as used herein refers to a collection of datasets. Particularly, the dataset pool herein refers to a collection comprisingdifferent types of datasets representing an outcome of anamplification reactionusing a signal-generating means for a target analyte. The different types of datasets in the dataset pool are obtained from the amplification reaction of step (a). The dataset pool may comprise 2, 3, 4, 5, 6, 7, or more different types of datasets.

According to an embodiment of the present invention, the datasets in the dataset pooleach comprises an amplification cycle number and a signal value at the amplification cycle number.

The term "cycle" as used herein refers to a unit of changes of conditions in a plurality of measurements accompanied with constant changes of conditions. The constant changes of conditions include an increase or decrease in reaction time, reaction number, concentration, pH, replication number of a measured subject (*e.g.,* nucleic acid molecule). In addition, the cycle may comprise a unit which is mathematically processed from the unit of changes of conditions.

As one example, when a substrate decomposition capacity by an enzyme is analyzed depending on concentrations of the substrate, a plurality of measurements for the decomposition capacity by the enzyme is carried out with varying substrate concentrations. The increases in the substrate concentration may correspond to the changes of conditions and a unit of the increases may correspond to a cycle.

As another example, an isothermal amplification allows for a plurality of measurements for a sample in the course of reaction time under isothermal conditions and the reaction time may correspond to the changes of conditions and a unit of the reaction time may correspond to a cycle.

As still another example, when a first derivative is obtained from the dataset of isothermal amplification reaction of nucleic acid, a difference between reaction times may be correspond to the changes of conditions and a unit of the difference between reaction times may be correspond to a cycle.

More particularly, when repeating a series of reactions or repeating a reaction with a time interval, the term "cycle" refers to a unit of the repetition.

For example, in a polymerase chain reaction (PCR), a cycle refers to a reaction unit comprising denaturation of a target molecule, annealing (hybridization) between the target molecule and primers and primer extension. The increases in the repetition of reactions may correspond to the changes of conditions and a unit of the repetition may correspond to a cycle.

The term "signal value" as used herein means a signal value (*e.g.,* signal intensity) actually measured at an amplification cycle, or a modified value thereof. The modified value may include a mathematically processed value of the actually measured signal value (*i.e.,* a signal value in a raw dataset). Examples of the mathematically processed value of the actually measured signal value may include logarithmic values and derivatives of measured signal values. The derivatives of measured signal values may include multi-derivatives.

The cycle number and signal value as described above constitute a data point to be explained below.

The term "data point" as used herein means a coordinate value comprising a cycle number and a signal value at the cycle. The term "data" as used herein means all information constituting a dataset. For instance, an amplification cycle number and a signal value are data, respectively.

Data points obtained by the amplification reactionusing the signal-generating means may be plotted with coordinate values in a rectangular coordinate system. In the rectangular coordinate system, the X-axis represents cycles of the amplification reaction and the Y-axis represents signal valuesmeasured or processed at the cycles.

The term "dataset" as used herein refers to a set of data points. For example, the dataset may be a set of data points obtained directly from an amplification reaction in the presence of a signal-generating means, or a modified dataset obtained by modifying such dataset. The dataset may be a portion or all of the data points obtained bythe amplification reaction or the modified data points thereof. The dataset may be plotted to obtain an amplification curve.

In particular, the term "two or more different types of datasets" as used herein means that the two or more datasets obtained are different in their types from each other. The different types of datasets mean that some or all of data points corresponding to each other between the datasets are substantially different from each other. For instance, the different types of datasets mean that data points corresponding to each other are different in terms of units or magnitudes of data components (*e.g.*, cycle number and signal value). The difference intypes of two datasets depends upon the manner by which the datasets are obtained. Where two datasets were obtained in the same manner, the datasets may be considered as the same type. In other words, where two datasets were obtained in the same manner, the datasets may be considered as the same type, although some or all of corresponding data points are different from each other. Likewise, where two datasets are obtained in different manners, the datasets may be considered as different types of datasets, although some or all of corresponding data points are identical to each other.In addition, as the term "the different types of datasets" used herein is intended to encompass datasets obtained in different manners from one amplification, two or more datasets obtained in the same manner from two or more amplification reactions do not fall within the scope of the term. For example, when a first raw dataset is obtained by performing anamplification reaction and a second raw dataset is obtained by re-performing anamplification reaction, theyare not considered asdifferent types of datasets, even thoughthere may be some different data points between the two raw datasets due to possible experimental errors or variations.A raw dataset and one or more datasets obtained by various mathematical processings of the raw dataset may represent the different types of datasets.

According to an embodiment of the present invention, the different types of datasets may comprise a raw dataset. The raw dataset may comprise an amplification cycle number and a signal value at the amplification cycle number.

The term "raw dataset" as used herein refers to a set of data points (including cycle numbers and signal values) obtained directly from an amplification reaction. The raw dataset means a set of non-processed data points which are initially received from a device for performing a real-time PCR (*e.g.*, thermocycler, PCR machine or DNA amplifier). In an embodiment of the present invention, the raw dataset may include a raw dataset understood conventionally by one skilled in the art. In an embodiment of the present invention, the raw dataset may include a dataset prior to processing. In an embodiment of the present invention, the raw dataset may include a dataset which is the basis for the mathematically processed datasets as described herein. In an embodiment of the present invention, the raw dataset may include a dataset not subtracted by a baseline (no baseline subtraction dataset). The raw dataset may be outputtedfrom a real-time PCR machine and recorded and stored in Microsoft™Excel format *etc.*

According to an embodiment of the present invention, the different types of datasets may comprise one or more mathematically processed datasets of the raw dataset.

The term "mathematically processed dataset (of the raw dataset)" as used herein encompasses a dataset obtained primarily by mathematically processing the raw dataset, as well as a dataset obtained by mathematically re-processing theprimarily mathematic-processed dataset. For example, the term may include datasets obtained by mathematically processing the raw dataset in a various ways such as derivatives of the raw dataset such as a first order derivative, a second order derivative, a third order derivative and a higher order derivative.

According to an embodiment of the present invention, the mathematically processed dataset comprises mathematically processed signal values which are processed from the signal values of the raw dataset using a particular calculation or function.

The term used herein "calculation" in conjunction with dataset or signal value refers to a mathematical process for converting one or more inputs (data) into one or more results (outcomes). For example, signal values (*e.g.*, RFUs (relative fluorescence units)) obtained by a real-time PCR may be mathematically processed into logarithmic values via the calculation.

The term used herein "processing by a function"in conjunction with dataset or signal value refers to a mathematical processing by relation between a set of inputs (data) and a set of outputs (data). For example, the raw dataset is mathematically processed by a linear regression analysis in order to calculate a slope of a baseline region. Alternatively, the raw dataset may be mathematically processed by a liner regression analysis in order to calculate a derivative of the raw dataset.

According to an embodiment of the present invention, the mathematically processed dataset comprises an $m^{th}$order change amount ($m^{th}$ order rate of change) of signal values of the raw dataset, wherein m is an integer of 1 or more. Particularly, m is an integer of 1-10, 1-5, 1-3, or 1-2. The $m^{th}$ change amount of signal values may be an $m^{th}$ order derivative (slope) of signal values. The $m^{th}$ order derivative may be obtained from slope valuesof the linear regression lines (for multiple cycles of an amplification reaction) given bya linear regression analysis. Such a mathematically processed dataset may be referred to as "slope dataset", and if plotted, as "slope curve".

Particularly, the linear regression analysis used in the present invention is carried out in accordance with a least square method. The least square method is represented by the following mathematical equation I:

<Equation I>

$$m = \frac{\sum_{i=I-a}^{I+b} (x_i - \bar{x})(y_i - \bar{y})}{\sum_{i=I-a}^{I+b} (x_i - \bar{x})^2}$$

wherein,

$$\overline{x} = \frac{\sum_{i=I-a}^{I+b} x_i}{n}, \quad \overline{y} = \frac{\sum_{i=I-a}^{I+b} y_i}{n} ;$$

I is a cycle of a data point whose slope is to be calculated;
m is a slope of a data point at $i^{th}$ cycle;
Xi is a cycle of $i^{th}$ cycle;
Yi is a signal value measure at $j^{th}$ cycle;
n is a+b+1; and
a and b independentlyrepresent an integer of 0-10 with a proviso that a is less than I, a+b+1 ranges from 2 to the number of data points of the raw datasets, and I+b is less than the number of data points of the raw data sets.

The "a+b+1" is the number of data points used for calculating a slope at$i^{th}$cycle, called as LSMR (Linear Squares Method Range). The "a" is a value for calculating a minimum cycle among a set of data points used for calculating a slope at$i^{th}$cycle. The "b" is a value for calculating a maximum cycle. The number of data points refers to the data points obtained from the overall reaction, corresponding to the maximum cycle value (times) of the plotted curve. The "a" and "b" are independently represent an integer of 0-10, particularly 1-5, and more particularly 1-3.

According to an embodiment of the present invention, the mathematically processed dataset is a dataset comprising signal values subtracted by a baseline determined to remove background signal values of the raw dataset. Such mathematically processed dataset may be referred herein to as "baseline subtracted dataset", and if plotted, as "baseline subtracted curve".

The baseline subtracted dataset may be obtained by a number of methods known in the art (*e.g.,* U.S. Patent No. 8,560,247). For instance, the dataset may be obtained by a process comprising the steps of: (i) determining a baseline region by determining both a start-point cycle and an end-point cycle of the baseline region using the raw dataset; (ii) establishing a function for a best-fit line of the baseline region using at least two data within the determined baseline region; and (iii) obtaining a dataset by subtracting values of the function of the best-fit line from the values of the signals of the raw dataset.

Particularly, the baseline subtracted dataset may be obtained by (i) determining a baseline region as a region ranging from $1^{th}$, $2^{nd}$, $3^{rd}$, $4^{th}$, or $5^{th}$cycle to a cycle before amplification starts; (ii) calculating an equation ofa linear regression line for cycles in the baseline region; and (iii) subtracting values of the signals at corresponding cycles calculated by the equation of a linear regression line from values of the signal values measured at each cycle.

According to an embodiment of the present invention, the dataset pool provided in step (b) comprises at least one dataset selected from the group consisting of a raw dataset and one or more mathematically processed datasets thereof.

According to an embodiment of the present invention, the dataset pool provided in step (b) comprises a raw dataset and a mathematically processed dataset thereof.

According to an embodiment of the present invention, the dataset pool provided in step (b) comprises a raw dataset and two or more mathematically

More specifically, the dataset pool provided in step (b) comprises: (i) a raw dataset; (ii) a mathematically processed dataset comprising an $m^{th}$order change amount of signal values of the raw dataset, wherein m is an integer of 1 or more; and (iii) a mathematically processed dataset comprising signal values subtracted by a baseline determined to remove background signal values of the raw dataset.

In addition to the datasets described above, the dataset pool provided in step (b) may further comprise other types of datasets which are obtainable from the amplification reaction using the signal-generating means for the target analyte. The other types of datasets included in the dataset pool may be various types of datasets known in the art. The datasetsincluded in the dataset pool are not limited, as long as they can be used to determine the presence or absence of a target analyte in a sample. The datasets included in the dataset pool may be obtained and provided via a program, software or algorithm. For example, various types of datasets known in the art to be used within the program, software or algorithm are inputted in advance and a user can then select (check), for example via check boxes, types of datasets desired to be included in the dataset pool. The program, software and algorithm may be designed such that the selected datasets are generated from an outcome of the amplification reaction by conventional methods and the generated datasets are provided (displayed) in step (b).

In the present step, there is no limitation to time-points at which the different types of datasets are provided. The different types of datasets may be provided almost simultaneously. The different types of datasets may be provided at different time points, for example, sequentially. Even if the datasets are provided at different time points, it is recognized by the one skilled in the art that a dataset pool is finally provided in the step.

At least one of the different types of datasets may be obtained or generated at a time point as needed, and then provided.

The present method may further comprise the step of selecting types of datasets to be used for determining the presence or absence of the target analyte.

(ii) Provision of determinative factors

Secondly, two or more determinative factors for determining the presence or absence of the target analyte are provided in the step. The determinative factors are obtained by evaluating with predefined criteria two or more datasets selected from the dataset pool; wherein each determinative factor is obtained from each type of dataset; wherein each of the criteria is predefined for each type of the datasets.

The expression "the determinative factors are obtained by evaluating two or more datasets selected from the dataset pool" is intended to mean that the determinative factors are not necessarily obtained by evaluating all datasets within the dataset pool. In other words, the determinative factors in step (b) may be in the number equal to or less than that of the datasets in the dataset pool. For example, it is possible that the number of the datasets in the dataset pool provided in step (b) is 5 and the number of the determinative factors provided in step (b) is 5, 4, 3, or 2.

The method may further comprise a step of selecting datasets to be evaluated among the datasets within the dataset pool. Afterwards, the selected datasets only may be used to provide the determinative factors. The selection of datasets to be evaluated may be implemented by a particular program, software, or algorithm.

The datasets to be selected for providing the determinative factors in step (b) may be all of the datasets contained in the dataset pool. In this case, the selection step may comprise selecting all datasets within the dataset pool.

The datasets to be selected for providing the determinative factors in step (b) may be some of the datasets contained in the dataset pool. In this case, the selection step may comprise selecting some datasets within the dataset pool.

The datasets to be selected for providing the determinative factors in step (b) comprise at least one dataset selected from the group consisting of a raw dataset and one or more mathematically processed datasets thereof.

The datasets to be selected for providing the determinative factors in step (b) comprise a raw dataset and a mathematically processed dataset thereof.

The datasets to be selected for providing the determinative factors in step (b) comprise a raw dataset and two or more mathematically processed datasets thereof.

More specifically, the datasets to be selected for providing the determinative factors in step (b) comprise: (i) a raw dataset; (ii) a mathematically processed dataset comprising an $m^{th}$ order change amount of signal values of the raw dataset, wherein m is an integer of 1 or more; and (iii) a mathematically processed dataset comprising signal values subtracted by a baseline determined to remove background signal values of the raw dataset.

The selection of two or more datasets to be evaluated for providing the determinative factors in step (b) may depend upon a variety factors, e.g., including utility of a particular dataset in the determination of the presence or absence and preference toward a particular dataset.

The term "determinative factor" as used herein refers to a preliminary reference prior to final determination of the presence or absence of a target analyte in a sample, which is obtained by evaluating a dataset based upon a criterion. The term "determinative factor" refers to a considerable factor in finally determining the presence or absence of a target analyte in a sample.

As described above, a determinative factor is obtained from one dataset. In other words, one dataset provides one determinative factor. Accordingly, the two or more determinative factors herein are those obtained from two or more different types of datasets.

According to an embodiment of the present invention, as defined by the appended claims the determinative factor is represented by (i) a positive determinative factor representing the probability of the presence of the target analyte or a negative determinative factor representing the probability of the absence of the target analyte; or (ii) a predeterminative value to be used in the determination of the presence or absence of the target analyte; wherein the predeterminative value does not represent the probability of the presence or absence of the target analyte. Particularly, the determinative factor is represented by either a positive determinative factor representing the probability of the presence of the target analyte or a negative determinative factor representing the probability of the absence of the target analyte.

The determinative factors may be classified into the following two types:
The first type of the determinative factor includes those capable of representing the probability of the presence or absence of the target analyte by themselves.

Particularly, the determinative factor is represented by either a positive determinative factor or a negative determinative factor. The positive determinative factor and the negative determinative factor are indicative values, or yes/no type indications.

The term "indicative value" as used herein refers to a numerical value representing the probability of the presence or absence of a target analyte, which is considered with comparing with a reference value. The indicative value may be a numerical value representing the probability of the presence of the target analyte, for example, a numerical value exceeding a predefined threshold, or a numerical value representing the probability of the absence of the target analyte, for example, a numerical value not more than a predefined threshold. For example, when a threshold for a particular dataset is set to "50", numerical values exceeding 50 (*e.g.,* 60, 70, *etc.*)may be positive determinative factors representing the probability of the presence of the target analyte, whereas numerical values not more than 50 (e.g., 40, 30, etc.) may be negative determinative factors representing the probability of the absence of the target analyte.

The term "yes/no type indication" as used herein refers to expressing the determinative factor as only two indications depending upon the probability of the presence or absence of the target analyte. The indication may include any symbol, sign, word and description. For example, when the determinative factor is represented by a positive determinative factor representing the presence of the target analyte, it may be expressed as "+", "Yes", "Y", "1", "Plus"or "P (positive)". When the determinative factor is represented by a negative determinative factor representing the absence of the target analyte, it may be expressed as "-", "No", "N", "0", "Minus" or"N (negative)". The "yes/no type indication" may be expressed in variousfashions.

The term "probability" is used herein to refer to an instance where a possibility of either the presence or absence of the target analyte exceeds 50%, 60%, 70%, 80% or 90%. Accordingly, the expression "representing the probability of the presence of the target analyte" means that a possibility of the presence of the target analyte exceeds 50%, 60%, 70%, 80% or 90%, and the expression "representing the probability of the absence of the target analyte" means that a possibility of the absence of the target analyte exceeds 50%, 60%, 70%, 80% or 90%.

One of the characteristics of the present invention lies in that the presence or absence of the target analyte can be finally determined, even using determinative factors expressed simply as a yes/no type indication, with more improved accuracy than conventional methods. According to conventional methods, the determination of the presence or absence of a target analyte has been assessed by using only one dataset, which corresponds to using one determinative factor. In contrast, the present method employs two or more determinative factors, allowing for more accurate determination.

The second type of the determinative factorincludes a predeterminative value to be used in the determination of the presence or absence of the target analyte; wherein the predeterminative value does not represent the probability of the presence or absence of the target analyte. The term "predeterminative value" as used herein refers to a value being used in the final determination, but beingirrelevant to the presence or absence of the target analyte by itself. For example, when the predeterminative value is a numerical value such as "100", this numerical value *per se*may not represent the probability of the presence or absence of the target analyte, but be used to finally determine the presence or absence of the target analytewith considering all provided determinative factors. The predeterminative value may be applied to a certain mathematical equation or calculation for obtaining a final value and the final value may be then evaluated based on a predefined criterion, indicating the presence or absence of the target analyte.

It will be appreciated by one of skill in the art that these determinative factors are merely illustrative and other determinative factors may be used instead.

The determinative factors as described above are obtained by evaluating with predefined criteria two or more datasets selected from the dataset pool.

The term "criterion (or criteria)" as used herein in conjunction with evaluation of datasets refers to a basis for deducing a determinative factor, *e.g.,* a positive determinative factor or a negative determinative factor, or a predeterminative value from a dataset. In addition, the "criterion" refers to a basis which can be used to identify whether a dataset has a certain characteristic representing the presence or absence of the target analyte.

As one example, the "criterion" may refer to an indicator or item which can be applied to a dataset for differentiating between a positive (presence of target analyte) sample and a negative (absence of target analyte) sample. Specifically, when a signal value is used as an indicatorto differentiate between a positive sample and a negative sample, the signal value may be the "criterion".

As another example, the "criterion" may refer to a numerical value (*e.g.,* threshold) which can be applied to a dataset for differentiating between a positive sample and a negative sample. Specifically, when a numerical value "200" is established as a value for differentiating a positive sample and a negative sample, the numerical value "200" may be the "criterion".

As further example, the "criterion" may refer to a condition relative to (or with reference to) a particular value (*e.g.,* threshold) for differentiating a positive sample and a negative sample. Specifically, the expression "when exceeding a numerical value 200, the sample corresponds to a positive sample" may be the "criterion".

The criterion may vary depending upon the types of the datasets.

Specific examples of the criterion include, without limitation, a ratio threshold of signal values, a $R^2$ threshold in linear regression, a threshold of signal change amount, and a signal threshold. The criterion may be defined by iterative experimentation, may be selected from values known in the art, or may be defined using a reference sample (control sample). It would be recognized by one of skill in the art that other appropriate criteria may be used instead of the exemplary criteria as described above.

The criterion may be defined from a ratio of a signal value at a cycle to a signal value at another cycle. Generally, in case of positive samples, a signal value at an end-point cycle is greater than a signal value at a start-point cycle, whereas in case of negative samples, a signal value at an end-point cycle is not greater than a signal value at a start-point cycle. Therefore, "1.0", a ratio of a signal value at an end-point cycle to a signal value at a start-point cycle, may be predefined as a criterion. Afterwards, a negative determinative factor is obtained from a dataset having the ratio of not more than 1.0, and a positive determinative factor is obtained from a dataset having the ratio of more than 1.0. In this case, the ratio of signal values "1.0" is a threshold serving as the criterion of step (b).

The criterion may be defined from a ratio of a mean signal value in a region to a mean signal value in another region. Generally, in case of positive samples, a mean signal value in an end region (*e.g.,* ranging from 45 cycle number to 50 cycle number) of an amplification reaction is greater than a mean signal value in a start region (*e.g.*, ranging from 1 cycle number to 5 cycle number), whereas in case of negative samples, a mean signal value in an end region is not greater than a mean signal value in a start region. Therefore, "1.0", a ratio of a mean signal value at an end region to a mean signal value at a start region, may be predefined as a criterion. Afterwards, a negative determinative factor is obtained from a dataset having the ratio of not more than 1.0, and a positive determinative factor is obtained from a dataset having the ratio of more than 1.0. In this case, the ratio of mean signal values "1.0" is a threshold serving as the criterion of step (b).

Some or all of the raw dataset is subjected to a linear regression fit, after which a negative determinative factor is obtained from a dataset having $R^2>0.99$ in the linear regression, and otherwise a positive determinative factor is obtained. In this case, the $R^2$ in the linear regression "0.99" is a threshold serving as the criterion of step (b).

Some or all of the raw dataset is subjected to a quadratic regression fit, after which a negative determinative factor is obtained from a dataset having $R^2>0.98$ in the quadratic regression, and otherwise a positive determinative factor is obtained. In this case, the $R^2$ in the quadratic regression "0.98" is a threshold serving as the criterion of step (b).

A signal change rate "40" is applied to a mathematically processed curve (slope curve) comprising an $m^{th}$ order change amount of signal values of the raw dataset, after which a negative determinative factor is obtained from a dataset having a peak value of not less than 40, and otherwise a positive determinative factor is obtained. In this case, the signal change rate "40" is a threshold serving as the criterion of step (b).

A threshold of signal value "150" is applied to a mathematically processed curve subtracted by a baseline (baseline subtracted curve), after which a positive determinative factor is obtained from a dataset having a maximum signal value of not less than 150, and otherwise a negative determinative factor is obtained. In this case, the signal value "150" is a threshold serving as the criterion of step (b).

As such, the criterion used to obtain a positive or a negative determinative factor may vary depending upon each type of the datasets.

In the present step, the evaluation of the datasets with the predefined criteria may be performed by employing as the criterion a threshold assigned to each type of the datasets for determining the presence or absence of the target analyte, as described above.

Alternatively, the evaluation of the datasets with the predefined criteria may be performed by employing as the criterion a plurality of thresholds assigned to each type of the datasets for determining the presence or absence of the target analyte. For example, the criterion predefined for each type of the datasets may comprise a plurality of sub-criteria, and the datasets may be evaluated with the sub-criteria, generating a plurality of sub-determinative factors. Then, the sub-determinative factors are assessed (*e.g.*, by calculation) to obtain a determinative factor for the dataset.

In the present step, there is no limitation to time-points at which the determinative factors are provided. The determinative factors may be provided almost simultaneously. The determinative factors may be provided at different time points, for example, sequentially. The determinative factors may be provided immediately after the datasets are provided, or may be provided separately from the provision of the datasets. As one example, a first dataset is provided and a first determinative factor for the first dataset is then provided, after which a second dataset is provided and a second determinative factor for the second dataset is then provided. As another example, a first dataset and a second dataset are provided, after which a first determinative factor for the first dataset and a second determinative factor for the second dataset are then provided.

At least one of the determinative factors may be obtained from a corresponding dataset at a time point as needed, and then provided.

The step (b) may comprise assessing at least one determinative factor among the provided determinative factors by using a criterion. The step (b) may comprise assessing a preceding determinative factor by using a criterion and then optionally providing a next determinative factor depending upon the assessment result.

**Step (c): Determining the presence or absence of target analyte(400)**

In the present step, the presence or absence of the target analyte in the sample is determined by using at least two of the determinative factors.

The expression "using at least two of the determinative factors" as used herein means that all determinative factors provided in step (b) are not necessarily used in step (c). In other words, some or all of the determinative factors provided in step (b) may be used in the determination of step (c). Particularly, the utilization of some of the determinative factors provided in step (b) for the determination in step (c) may be an embodiment of a "sequential determination approach" as described below.

In addition, the expression "using at least two of the determinative factors" as used herein refers to considering (or assessing) the at least two of the determinative factors in the determination of the presence or absence of the target analyte in the sample. In other words, the expression means that the at least two of the determinative factors are considerable factors for determining the presence or absence of the target analyte in the sample.

Furthermore, the expression "determining the presence or absence of the target analyte in the sample by using at least two of the determinative factors" is intended to encompass assessing the at least two of the determinative factors based on a predefined criterion and determining the presence or absence of the target analyte from the assessment results. Such assessment refers to identifying whether the determinative factors satisfy the predefined criterion.

In addition, the expression "determining the presence or absence of the target analyte in the sample by using at least two of the determinative factors" is intended to encompass all instances where the presence or absence of a target analyte may be finally determined by using at least two of the determinative factors, irrespective of how the determinative factors are assessed.

The step (c) comprise assessing the determinative factors used for the presence or absence determination sequentially in the steps of providing the determinative factors; wherein the at least two determinative factors are finally assessed for the presence or absence determination. Alternatively, the step (c) comprise assessing the determinative factors used for the presence or absence determination sequentially in the steps of providing the determinative factors; wherein a next determinative factor is provided depending upon the assessment result of a preceding determinative factor, and the at least two determinative factors is finally assessed for the presence or absence determination.

According to an embodiment of the present invention as defined by the appended claims the determinative factors used in step (c) comprise at least one determinative factor obtained by evaluating at least one dataset selected from the group consisting of a raw dataset and one or more mathematically processed datasets thereof.

According to an embodiment of the present invention, as defined by the appended claims the determinative factors used in step (c) comprise (i) a determinative factor obtained by evaluating a raw dataset; and (ii) a determinative factor obtained by evaluating a mathematically processed dataset of a raw dataset.

According to an embodiment of the present invention, as defined by the appended claims the determinative factors used in step (c) comprise (i) a determinative factor obtained by evaluating a raw dataset; and (ii) two or more determinative factors obtained by evaluating two or more mathematically processed datasets of a raw dataset.

More specifically, the determinative factors used in step (c) comprise: (i) a determinative factor obtained by evaluating a raw dataset; (ii) a determinative factor obtained by evaluating a mathematically processed dataset comprising an $m^{th}$ order change amount of signal values of the raw dataset, wherein m is an integer of 1 or more; and (iii) a determinative factor obtained by evaluating a mathematically processed dataset comprising signal values subtracted by a baseline determined to remove background signal values of the raw dataset.

According to an embodiment of the present invention, as defined by the appended claims in case that the determinative factor is represented by a positive determinative factor or a negative determinative factor, the determination of the presence or absence of the target analyte may be performed as follows: when the determinative factors in step (c) are all positive determinative factors, the presence of the target analyte in the sample is determined, and when at least one of the determinative factors in step (c) is a negative determinative factor, the absence of the target analyte in the sample is determined. For example, when three determinative factors used in step (c) are (+), (+) and (+), the presence of the target analyte in the sample is determined, and otherwise the absence of the target analyte in the sample is determined.

Particularly, the determination of the presence or absence of the target analyte may be performed mainly by two approaches: collective (or parallel) determination and sequential (or serial) determination.

(i) Collective (or parallel) determination approach

The term "collective determination approach" or "parallel determination approach" refers to an approach to take into account the determinative factors in step (b) collectively or simultaneously for determining the presence or absence of the target analyte in the sample.

The collective determination approach is illustrated in Fig. 2. As depicted in Fig. 2, three datasets are obtained from an amplification reaction (200) and three determinative factors are obtained from the datasets (300). Afterwards, the three determinative factors are collectively assessed to determine the presence or absence of a target analyte in a sample (400).

The determination of the presence or absence of the target analyte in the sample is performed by assessing the two or more determinative factors collectively with a predefined presence or absence criterion. The collective assessment of the two or more determinative factors with a predefined presence or absence criterion is performed by identifying whether a combination of the two or more determinative factors satisfies a predefined presence or absence criterion.

In case that the determinative factors are represented by positive determinative factors or negative determinative factors, the combination of the two or more determinative factors may include a collection of all the determinative factors used in step (c). For example, a combination of three determinative factors may include a collection of the three determinative factors such as a collection of a positive determinative factor, a positive determinative factor and a negative determinative factor.

In addition, in case that the determinative factors are represented by predeterminative values, the combination of the two or more determinative factors may include a value derived from a calculation of the predeterminative values.

The terms "presence criterion" or "absence criterion" as used herein refer to a basis for determining the presence or absence of the target analyte in the sample by using the combination of the two or more determinative factors.

Where the combination of the two or more determinative factors satisfies a predefined presence criterion, the presence of the target analyte in the sample is determined; where the combination of the two or more determinative factors satisfies a predefined absence criterion, the absence of the target analyte in the sample is determined. The presence criterion and the absence criterion may be set such that they may cover all possible combinations of the two or more determinative factors, or may be set such that they may not cover all possible combinations of the two or more determinative factors. When the presence criterion and the absence criterion are set to cover all possible combinations, one of the presence and the absence is necessarily determined. In contrast, when the presence criterion and the absence criterion are set not to cover all possible combinations, none of the presence and the absence may be determined in some combinations. In this case, other determinations than the presence or the absence could be made, for example, determinations such as 'undetermined' or 're-test' may be made.

According to an embodiment of the collective determination approach, when the determinative factors are represented by positive determinative factors or negative determinative factors, the predefined presence or absence criterion may be defined by a proportion of a positive determinative factor or a negative determinative factor among a total of the determinative factors.

As one example, the predefined presence criterion may be a criterion defined by which a proportion of a positive determinative factor among a total of the determinative factors is more than 50%, more than 60%, more than 70%, more than 80%, more than 90%, or 100%. As another example, the predefined presence criterion may be a criterion defined by which a proportion of a negative determinative factor among a total of the determinative factors is less than 50%, less than 40%, less than 30%, less than 20%, less than 10%, or 0%.

As one example, the predefined absence criterion may be a criterion defined by which a proportion of a negative determinative factor among a total of the determinative factors is more than 50%, more than 60%, more than 70%, more than 80%, more than 90%, or 100%. As another example, the predefined absence criterion may be a criterion defined by which a proportion of a positive determinative factor among a total of the determinative factors is less than 50%, less than 40%, less than 30%, less than 20%, less than 10%, or 0%.

Particularly, the predefined presence criterion may be a criterion defined by which a proportion of a positive determinative factor among a total of the determinative factors is 100%, and the predefined absence criterion may be a criterion defined by which a proportion of a negative determinative factor among a total of the determinative factors is 100%.

Specifically, when all of the determinative factors used for the presence or absence determination are all positive, the presence of the target analyte in the sample is determined; and when at least one of the determinative factors is a negative determinative factor, the absence of the target analyte in the sample is determined. For example, when two of three determinative factors are positive determinative factors and one of three determinative factors is a negative factor, the absence of the target analyte in the sample is finally determined (*see* Fig. 2).

Alternatively, when the number of positive determinative factors among the determinative factors used for the presence or absence determination is greater than that of negative determinative factors, the presence of the target analyte in the sample may be finally determined; and when the number of negative determinative factors among the determinative factors used for the presence or absence determination is equal to or greater than that of positive determinative factors, the absence of the target analyte in the sample may be finally determined. For example, in an embodiment method using five determinative factors from five different types of datasets for the presence or absence determination, when three of five determinative factors are positive determinative factors and two of five determinative factors are negative determinative factors, the presence of the target analyte in the sample is finally determined.

According to an embodiment, when the determinative factors are represented by predeterminative values, the predefined presence or absence criterion may be defined by calculation of the predeterminative values.

As one example, the predefined presence criterion may be a criterion defined by which a value derived from calculation of the predeterminative values is greater than a predefined value, and the predefined absence criterion may be a criterion defined by which a value derived from calculation of the predeterminative values is equal to or less than a predefined value. According to the presence criterion and the absence criterion, the presence or absence of the target analyte in the sample may be finally determined. For example, when a value derived from calculation of three predeterminative values, "$p$" is greater than a predefined value "$q$", the presence of the target analyte in the sample may be finally determined, and when "$p$" is equal to or less than "$q$", the absence of the target analyte in the sample may be finally determined.

According to an embodiment of the collective determination approach, each of the determinative factors may be weighed. For example, if a determinative factor obtained by evaluating a particular dataset is more reliable in the determination of the presence or absence of the target analyte in the sample, the determinative factor may be more weighed.

(ii) Sequential (or serial) determination approach

The term "sequential determination approach" or "serial determination approach" as used herein refers to a stepwise approach to take into account the determinative factors sequentially according to their orders, for determining the presence or absence of the target analyte in the sample.

According to an embodiment of the sequential determination approach, the number and types of datasets to be evaluated are selected in advance, and analysis orders are then assigned to the datasets. In this case, the datasets except for a first-ordered dataset may be all generated and provided without considering whether they will be evaluated for analysis, or may be generated and provided when they are needed for analysis.

The sequential determination approach may be performed in various ways by one of skill in the art.

As one example, the sequential determination approach may be performed according to a "go or stop" fashion. Such approach assesses the two or more determinative factors sequentially. When a preceding determinative factor is assessed to satisfies a "stop" criterion, the presence or absence is immediately determined without considering a next determinative factor (stop); when preceding determinative factor is assessed tosatisfy a "go" criterion, a next determinative factor is then assessed (go).

According to anembodiment, the determination of the presence or absence of the target analyte is performed by assigning an order to the datasets selected from the dataset pool, and assessing the determinative factors having the same order as the corresponding datasets sequentially in the order with a predefined "go or stop" criterion. Specifically, the datasets selected from the dataset pool in step (b) may be assigned to an order, and the datasets are evaluated sequentially in the order to obtain the determinative factors. And then, the determinative factors having the same order as the corresponding datasets are assessed sequentially with a predefined "go or stop" criterion.

According to an embodiment,the determinative factors to be assessed may be selected in advance. The selected determinative factors may be all provided prior to the assessment. Alternatively, the determinative factors may be provided sequentially. Therefore, when a determinative factor at a certain order satisfies the "stop" criterion, a next determinative factor as well as a dataset from which the next determinative factor need not be obtained or provided.

The term "go or stop" criterion refers to a basis for determining whether a next determinative factor is considered or not, when the two or more determinative factors are used sequentially. In an embodiment of the present invention, the "go or stop" criterion is defined by whether a determinative factor is positive or negative and whether a next determinative factor is present or not.

The "go or stop" criterion used in the present invention may comprise a "go" criterion and a "stop" criterion.

The "stop" criterion refers to a criterion which directs to conclude that the influence of a determinative factor at a certain order on the determination of the presence or absence of the target analyte in the sample is highly significant compared withthose of other determinative factors and therefore the other determinative factors need not be assessed. In contrast, the "go" criterion refers to a criterion which directs to conclude that the influence of a determinative factor at a certain order on the determination of the presence or absence of the target analyte in the sample is not significant compared with other determinative factors and therefore the other determinative factors need be assessed.

According to an embodiment, the "go or stop" criterion comprises (i) a "stop" criterion defined by which a determinative factor is negative,(ii) a "stop" criterion defined by which a determinative factor is positive and a next determinative factor is no longer present, and (iii) a "go" criterion defined by which a determinative factor is positive and a next determinative factor is present; wherein when the determinative factor at a certain order satisfies the "stop" criterion defined by (i), the absence of the target analyte is determined; when it satisfies the "stop" criterion defined by (ii), the presence of the target analyte is determined; and when it satisfies the "go" criterion defined by (iii), a next determinative factor is then assessed.

One example of the sequential determination approach is depicted in Fig. 3. In the sequential determination approach, three determinative factors (*i.e.,* 1st, 2nd and 3rd determinative factors) all are provided in advance prior to analysis.

As illustrated in Fig. 3, a first determinative factor is assessed whether it satisfies the "go or stop" criterion. As the first determinative factor is positive ("+") and a next determinative factor (*e.g.,* 2nd determinative factor) is present, it satisfies the "go" criterion defined by (iii). Therefore, a next second determinative factor is assessed whether it satisfies the "go or stop" criterion. As the next second determinative factor is positive ("+") and a next determinative factor (*e.g.,* 3rd determinative factors) is present, it satisfies the "go" criterion defined by (iii). Therefore, a next third determinative factor is assessed whether it satisfies the "go or stop" criterion. As the next third determinative factor is positive ("+") and a next determinative factor is not present, it satisfies the "stop" criterion defined by (ii). Therefore, the presence of a target analyte in a sample may be determined.

Another example of the sequential determination approach is depicted in Fig. 4. In the sequential determination approach, three determinative factors (*i.e.,* 1st, 2nd and 3rd determinative factors) all are provided in advance prior to analysis.

As illustrated in Fig. 4, a first determinative factor is assessed whether it satisfies the "go or stop" criterion. As the first determinative factor is positive ("+") and a next determinative factor (*e.g.,* 2nd determinative factor) is present, it satisfies the "go" criterion defined by (iii). Therefore, a next second determinative factor is assessed whether it satisfies the "go or stop" criterion. As the next second determinative factor is negative ("-"), it satisfies the "stop" criterion defined by (i). Therefore, the absence of a target analyte in a sample may be determined without assessing a next determinative factor (*e.g.,* 3rd determinative factor).

As illustrated in Figs 3 and 4, the determinative factors to be assessed are selected and provided in advance prior to the analysis. However, since a next determinative factor need not be assessed when a preceding determinative factor satisfies the "stop" criterion, the next determinative factor(*e.g.*, 3rd determinative factor in Fig. 4) as well as a corresponding dataset need not be obtained and provided in advance.

According to the sequential determination approach, there may be any differences between the number of the determinative factors provided in step (b) and the number of the determinative factor used in step (c). It occurs when a preceding determinative factor satisfies a "stop" criterion and there is a remaining determinative factor to be assessed.

For the sequential determination approach, the number of datasets (*i.e.,* determinative factors) used in step (c) may vary depending upon whether a determinative factor at a certain order is assessed to satisfy either a "stop" criterion or a "go" criterion.

According to an alternative embodiment, the "go or stop" criterion comprises (i) a "stop" criterion defined by which a determinative factor is positive,(ii) a "stop" criterion defined by which a determinative factor is negative and a next determinative factor is no longer present, and (iii) a "go" criterion defined by which a determinative factor is negative and a next determinative factor is present; wherein when the determinative factor at a certain order satisfies the "stop" criterion defined by (i), the presence of the target analyte is determined; when it satisfies the "stop" criterion defined by (ii), the absence of the target analyte is determined; and when it satisfies the "go" criterion defined by (iii), a next determinative factor is then assessed.

## II. Storage Medium, Device and Computer Program

In other aspect of this invention, defined by the appended claims there is a provided a computer readable storage medium containing instructions to configure a processor to perform a method for determining the presence or absence of a target analyte in a sample by a multiple dataset analysis (MDA), the method comprising:

(a) receiving an outcome of an amplification reaction using a signal-generating means for the target analyte;
(b) providing (i) a dataset pool comprising different types of datasets representing an outcome of the amplification reaction and (ii) two or more determinative factors for determining the presence or absence of the target analyte; wherein thedeterminative factors are obtained by evaluating with predefined criteria two or more datasets selected from the dataset pool; wherein each determinative factor is obtained from each type of dataset; wherein each of the criteria is predefined for each type of the datasets; and
(c) determining the presence or absence of the target analyte in the sample by using at least two of the determinative factors.

In an another aspect of the invention, there is provided a computer program to be stored in a computer readable storage medium containing instructions to configure a processor to perform a method for determining the presence or absence of a target analyte in a sample by a multiple dataset analysis (MDA), the method comprising:

(a) receiving an outcome of an amplification reaction using a signal-generating means for the target analyte;
(b) providing (i) a dataset pool comprising different types of datasets representing an outcome of the amplification reaction and (ii) two or more determinative factors for determining the presence or absence of the target analyte; wherein thedeterminative factors are obtained by evaluating with predefined criteria two or more datasets selected from the dataset pool; wherein each determinative factor is obtained from each type of dataset; wherein each of the criteria is predefined for each type of the datasets; and
(c) determining the presence or absence of the target analyte in the sample by using at least two of the determinative factors.

Since the storage medium, the device and the computer program of the prevent invention defined by the appended claims are intended to perform the present methods in a computer, the common descriptions between them are omitted in order to avoid undue redundancy leading to the complexity of this specification.

The program instructions are operative, when performed by the processor, to cause the processor to perform the present method described above. The program instructions for performing the method for determining the presence or absence of a target analyte in a sample may comprise: (i) an instruction to receive an outcome of an amplification reaction using a signal-generating means for the target analyte; (ii) an instruction to provide a dataset pool and two or more determinative factors; and (iii) an instruction to finally determine the presence or absence of the target analyte in the sample by using at least two of the determinative factors.

The present method described above is implemented in a processor, such as a processor in a stand-alone computer, a network attached computer or a data acquisition device such as a real-time PCR machine.

The types of the computer readable storage medium include, without limitation, various storage medium such as CD-R, CD-ROM, DVD, flash memory, floppy disk, hard drive, portable HDD, USB, magnetic tape, MINIDISC, nonvolatile memory card, EEPROM, optical disk, optical storage medium, RAM, ROM, system memory and web server.

The datasets may be received through several mechanisms. For example, the datasets may be acquired by a processor resident in a PCR data acquiring device. The datasets may be provided to the processor in real time as the data points are being collected, or it may be stored in a memory unit or buffer and provided to the processor after the experiment has been completed. Similarly, the datasets may be provided to a separate system such as a desktop computer system via a network connection e.g., LAN, VPN, intranet and Internet) or direct connection {e.g., USB or other direct wired or wireless connection) to the acquiring device, or provided on a portable medium such as a CD, DVD, floppy disk, portable HDD or the like to a stand-alone computer system. Similarly, the datasets may be provided to a server system via a network connection {e.g., LAN, VPN, intranet, Internet and wireless communication network) to a client such as a notebook or a desktop computer system.

The instructions to configure the processor to perform the present invention defined by the appended claims may be included in a logic system. The instructions may be downloaded and stored in a memory module {e.g., hard drive or other memory such as a local or attached RAM or ROM), although the instructions can be provided on any software storage medium such as a portable HDD, USB, floppy disk, CD and DVD. A computer code for implementing the present invention may be implemented in a variety of coding languages such as C, C++, Java, Visual Basic, VBScript, JavaScript, Perl and XML. In addition, a variety of languages and protocols may be used in external and internal storage and transmission of data and commands according to the present invention.

In still further aspect of this invention, as defiend by the appended claims there is provided a device for determining the presence or absence of a target analyte in a sample by a multiple dataset analysis (MDA), the device comprising: (a) a computer processor; and (b) the computer readable storage medium as described above which is coupled to said computer processor.

According to an embodiment, the device further comprises a reaction vessel to accommodate the sample and signal-generating means, a temperature controlling means to control temperatures of the reaction vessel and/or a detector to detect signals at amplification cycles.

The computer processor may be prepared in such a manner that a single processor can do all performances as described above. Alternatively, the processor unit may be prepared in such a manner that several processors do all performances, respectively.

According to an embodiment, the processor may be embodied by installing software into conventional devices for detection of target nucleic acid molecules (*e.g.*, real-time PCR device).

The features and advantages of this invention will be summarized as follows:

(a) The present invention dramatically reduces errors (particularly false-positive errors) in the determination of the presence or absence of a target analyte in a sample, by using two or more datasets derived from an amplification reaction; and

(b) Where the present invention is performed by using the yes/no type indication, it exhibits enhanced accuracy and convenience compared with conventional methods.

The present invention will now be described in further detail by examples.

## EXAMPLES

**EXAMPLE 1**: **Acquisition of various datasets from amplification reaction and determination of determinative factors**

In order to evaluate the effectiveness of the multiple dataset analysis (MDA)of the present invention, the MDA using two or three datasets was compared with a single dataset analysis using only one dataset as a control.

Positive samples and negative samples, each containing a target nucleic acid sequence or not, were subjected to a real-time PCR to obtain three types of datasets as follows: (i) raw dataset; (ii) mathematically processed dataset I; and (iii) mathematically processed dataset II. Afterwards, each dataset was evaluatedwith a predefined criterion to obtain a positive determinative factor (representing the probability of the presence of the target nucleic acid sequence) ora negative determinative factor (representing the probability of the absence of the target nucleic acid sequence).

<u><1-1>Acquisition of raw dataset and determination of determinative factor</u>

The following samples were subjected to real-time PCR reactions: (i) Twenty-four (24) samples which had been analyzed to contain a target nucleic acid sequence of interest at various concentrations (positive samples); and (ii) one-hundred twenty-nine (129) samples which had been analyzed to contain no target nucleic acid sequence of interest (negative samples). The target nucleic acid sequence was a genomic DNA of *Campylobacter* spp. The reaction was conducted in the tube containing a positive sample or a negative sample, a downstream primer, an upstream primer, TaqMan probe, and Master Mix containing $MgCl_2$, dNTPs and *Taq* DNA polymerase; the tube containing the reaction mixture was placed in the real-time thermocycler (CFX96, Bio-Rad); the reaction mixture was denatured for 15 min at 95°C and subjected to 45 cycles of 10 sec at 95°C, 60 sec at 60°C, 30 sec at 72°C. Detection of the signal was performed at 72°C of each cycle.

The raw datasetsfor each sample wereobtained from the amplification reaction. The raw dataset was composed of acycle number and a relative fluorescence unit(RFU) at each cycle number. The RFUs were plotted against cycle numbers to obtain a plot (X-axis: cycle number; Y-axis: RFU). The plot was named as "non-baseline subtracted curve".

Subsequently, for each non-baseline subtracted curve, the following Equation II was used to calculate a ratio of RFU at the end-point cycle (cycle 45) to RFU at the start-point cycle (cycle 1).

<Equation II>

Ratio of signal values = (RFU at cycle 45) / (RFU at cycle 1)

Then, a positive determinative factor was obtained fora samplehaving the ratio of not less than a predefinedratio threshold, whereas a negative determinative factor was obtained for a samplehaving the ratio belowthethreshold. In this Example, the ratio threshold was set to 1.0.

<u><1-2>Acquisition of mathematically processed dataset I and determination of its determinative factor</u>

The non-baseline subtracted curves in Example <1-1> were each used to calculate the slope of a fluorescent value at each cycle by using a least square method as known in the art. The calculated slopes were plotted against cycle numbers to obtain a so-called "slope curve" as one of mathematically processed datasets.

Then, a predefinedslope thresholdwas applied to each of the slope curves. A positive determinative factor was obtained fora sample having the slopes of not less than the threshold at all cycles, whereas a negative determinative factor was obtainedfora sample having the slopes of below the threshold at all cycles. In this Example, the slope threshold was set to 40.

<u><1-3>Acquisition of mathematically processed dataset II and determination of its determinative factor</u>

The non-baseline subtracted curve in Example <1-1> was used to determine a baseline and then the baseline was subtracted from the non-baseline subtracted curve to obtain a so-called "baseline subtracted curve". Specifically, a region ranging from 3th cycle to a cycle before an amplification signal significantly increaseswas set as a baseline, and a linear regression equation was derived from signal values at cycles within the range. Afterwards, the signal values measured at the cycles were subtracted by the signal values calculated at the cyclesby the linear regression equation to obtain the baseline subtracted curve.

A predefinedsignal threshold was applied to each of the baseline subtracted curves. A positive determinative factor was obtained for a sample having the signal values of not less than the threshold at all cycles, whereas a negative determinative factor was obtained for a sample having the signal values of below the threshold at all cycles. In this Example, the signal threshold was set to 200.

**EXAMPLE 2: Determination of the presence or absence of target nucleic acid sequence using a single dataset**

In the Example, the presence or absence of the target nucleic acid sequence was determined by using a single dataset for each sample.

Specifically,a single datasetamong the datasets described in Examples <1-1>, <1-2> and <1-3> for a sample was evaluated with a predefined criterion to obtain a determinative factor. When the determinative factor was evaluated to be a positive determinative factor, the presence of the target nucleic acid sequence was finally determined, whereas when the determinative factor was evaluated to be a negative determinative factor, the absence of the target nucleic acid sequence was finally determined.

The resultsfor the non-baseline subtracted curves of Example <1-1> are shown in Table 1.

**TABLE 1**

| Sample | Non-Baseline Subtracted Curve | | | Error Rate (%) | |
|---|---|---|---|---|---|
| | Error | Normal | Total | False Positive | False Negative |
| Negative | 39 | 90 | 129 | 25.5% (39/153) | 0.0% (0/153) |
| Positive | 0 | 24 | 24 | | |

As shown in Table 1, 39 samples among total 129 negative samples were erroneouslyassessed as positive samples containing the target nucleic acid sequence. In contrast, all of 24 positive samples were non-erroneouslyassessed as positive samples. It was shown that a false-positive error rate and a false-negative error rate for a total of 153 samples were 25.5% (39/153) and 0.0% (0/153), respectively.

The resultsfor the slope curves of Example <1-2> are shown in Table 2.

**TABLE 2**

| Sample | Slope Curve | | | Error Rate (%) | |
|---|---|---|---|---|---|
| | Error | Normal | Total | False Positive | False Negative |
| Negative | 9 | 120 | 129 | 5.9% (9/153) | 0.0% (0/153) |
| Positive | 0 | 24 | 24 | | |

As shown in Table 2, 9 samples among total 129 negative samples were erroneouslyassessed as positive samples containing the target nucleic acid sequence. In contrast, all of 24 positive samples were non-erroneouslyassessed as positive samples. Therefore, it was shown that a false-positive error rate and a false-negative error rate for a total of 153 samples were 5.9% (9/153) and 0.0% (0/153), respectively.

The resultsfor the baseline subtracted curves of Example <1-3> are shown in Table 3.

**TABLE 3**

| Sample | Baseline Subtracted Curve | | | Error Rate (%) | |
|---|---|---|---|---|---|
| | Error | Normal | Total | False Positive | False Negative |
| Negative | 8 | 121 | 129 | 5.2% (8/153) | 0.0% (0/153) |
| Positive | 0 | 24 | 24 | | |

As shown in Table 3, 8 samples among total 129 negative samples were erroneouslyassessed as positive samples containing the target nucleic acid sequence. In contrast, all of 24 positive samples were non-erroneouslyassessed as positive samples. Therefore, it was shown that a false-positive error rate and a false-negative error rate for a total of 153 samples were 5.2% (8/153) and 0.0% (0/153), respectively.

It was found in the all experimental results that all positive samples werenon-erroneously assessed, but a considerable number of negative samples were erroneously assessed (occurrence of false positive error), demonstrating that the utilization of only a single dataset is very likely to result in false negative errors in the determination of the presence or absence of target nucleic acid sequences in samples.

**EXAMPLE 3: Determination of the presence or absence of target nucleic acid sequence using two datasets**

Two datasets for each sample were evaluated with predefined criteria to obtain two determinative factors, and the presence or absence of the target nucleic acid sequence was then determined by using several combinations of the two determinative factors as follows: (i) a combination of determinative factors in Examples <1-1> and <1-2>; (ii) a combination of determinative factors in Examples <1-2> and <1-3>; and (iii) a combination of determinative factors in Examples <1-1> and <1-3>.

In such combinations, when all of the two determinative factors were positive determinative factors, the presence of the target nucleic acid sequence was finally determined for the sample, and when any one of the two determinative factors was a negative determinative factor, the absence of the target nucleic acid sequence was finally determined for the sample.

The resultsfor the non-baseline subtracted curves of Example <1-1> and the slope curves of Example <1-2> are shown in Tables 4 and 5.

### TABLE 4

| Case | Non-Baseline subtracted curve | Slope curve | Sample | |
|------|------|------|------|------|
| | | | Negative | Positive |
| 1 | - | - | 81 | 0 |
| 2 | + | - | 39 | 0 |
| 3 | - | + | 9 | 0 |
| 4 | + | + | 0 | 24 |
| | Total | | 129 | 24 |

### TABLE 5

| Sample | Multipe Dataset Analysis | | | | Error Rate (%) | |
|------|------|------|------|------|------|------|
| | Case | Error | Normal | Total | False Positive | False Negative |
| Negative | Case 1~3 | 0 | 129 | 129 | 0.0% (0/153) | 0.0% (0/153) |
| Positive | Case 4 | 0 | 24 | 24 | | |

As shown in Table 4, all of 129 negative samples were non-erroneouslyassessed as negative samples as they were evaluated to correspond to Cases 1 to 3 in which any one of the two determinative factors is a negative determinative factor. And, all of 24 positive samples were non-erroneously assessed as positive samples as they were evaluated to correspond to Case 4 in which both of the two determinative factors are positive determinative factors. Therefore, as shown in Table 5, it was found that a false-positive error rate and a false-negative error rate for a total of 153 samples were 0.0% (0/153) and 0.0% (0/153), respectively. The results indicate that Cases 2 and 3 to be erroneously evaluated by the single dataset analysis can be evaluated with no false error by the multiple dataset analysis of the present invention.

The results for the slope curves of Example <1-2> and the baseline subtracted curves of Example <1-3> are shown in Tables 6 and 7.

### TABLE 6

| Case | Slope curve | Baseline subtracted curve | Sample | |
|------|------|------|------|------|
| | | | Negative | Positive |
| 1 | - | - | 116 | 0 |
| 2 | + | - | 5 | 0 |
| 3 | - | + | 4 | 0 |
| 4 | + | + | 4 | 24 |
| | Total | | 129 | 24 |

**TABLE 7**

| Sample | Multipe Dataset Analysis | | | | Error Rate (%) | |
|---|---|---|---|---|---|---|
| | Case | Error | Normal | Total | False Positive | False Negative |
| **Negative** | **Case 1~3** | **4** | **125** | **129** | **2.6% (4/153)** | **0.0% (0/153)** |
| **Positive** | **Case 4** | **0** | **24** | **24** | | |

As shown in Table 6, 129 negative samples all were non-erroneously assessed as negative samples as they were evaluated to correspond to Cases 1 to 3 in which any one of the two determinative factors is a negative determinative factor. Four negative samples among total 129 negative samples were erroneously assessed as positive samples (*i.e.,* false positive error) as they were evaluated to correspond to Case 4 in which both of the two determinative factors are positive determinative factors. All of 24 positive samples were non-erroneously assessed as positive samples as they were evaluated to correspond to Case 4. Therefore, as shown in Table 7, it was found that a false-positive error rate and a false-negative error rate for a total of 153 samples were 2.6% (4/153) and 0.0% (0/153), respectively. The results address that the multiple dataset analysis of the present invention is superior to the single dataset analysis in eliminating false positive errors.

The results for the non-baseline subtracted curves of Example <1-1> and the baseline subtracted curves of Example <1-3> are shown in Tables 8 and 9.

**TABLE 8**

| Case | Non-baseline subtracted curve | Baseline subtracted curve | Sample | |
|---|---|---|---|---|
| | | | Negative | Positive |
| 1 | - | - | 84 | 0 |
| 2 | + | - | 37 | 0 |
| 3 | - | + | 6 | 0 |
| 4 | + | + | 2 | 24 |
| | Total | | 129 | 24 |

**TABLE 9**

| Sample | Multipe Dataset Analysis | | | | Error Rate (%) | |
|---|---|---|---|---|---|---|
| | Case | Error | Normal | Total | False Positive | False Negative |
| **Negative** | **Case 1~3** | **2** | **127** | **129** | **1.3% (2/153)** | **0.0% (0/153)** |
| **Positive** | **Case 4** | **0** | **24** | **24** | | |

As shown in Table 8, 127 negative samples among total 129 negative samples were non-erroneously assessed as negative samples as they were evaluated to correspond to Cases 1 to 3 in which any one of the two determinative factors is a negative determinative factor. Two negative samples among total 129 negative samples were erroneously assessed as positive samples as they were evaluated to correspond to Case 4 in which both of the two determinative factors are positive determinative factors. All of 24 positive samples were non-erroneously assessed as positive samples as they were evaluated to correspond to Case 4 in which both of the two determinative factors are positive determinative factors. Therefore, as shown in Table 9, it was found that a false-positive error rate and a false-negative error rate for a total of 153 samples were 1.3% (2/153) and 0.0% (0/153), respectively. The results address that the multiple dataset analysis of the present invention is superior to the single dataset analysis in eliminating false positive errors.

In the above-described results, the multiple dataset analysis of the present invention was shown to provide non-erroneous assessment for all positive samples while it provided erroneous assessment for a small number of negative samples (*i.e.,* false positive error). It is noteworthy that such false-positive error rate of the multiple dataset analysis is much lower than that of the single dataset analysis as shown in Example 2. Accordingly, it would be clearly realized that the multiple dataset analysis using multiple datasets (*e.g.,* two datasets) can greatly reduce the occurrence of false-positive errors.

**EXAMPLE 4: Determination of the presence or absence of target nucleic acid sequence using three datasets**

The multiple dataset analysis according to the present invention may be carried out in two different approaches:

(i) collective determination approach, in which each of datasets for a sample is evaluated with a predefined criterion to obtaina positive determinative factor associated with the presence of the target nucleic acid sequence or a negative determinative factor associated with the absence of the target nucleic acid sequence, and the determinative factors are then assessed collectively to finally determine the presence or absence of the target nucleic acid sequence in the sample; and

(ii) sequential determination approach, in which the determinative factors for datasets for a sample are assessed sequentially to finally determine the presence or absence of the target nucleic acid sequence in the sample; wherein when a preceding determinative factor is a negative determinative factor (when a preceding determinative factor satisfies a "stop" criterion), the absence of the target nucleic acid sequence is finally determined without considering a next determinative factor;when a preceding determinative factor is a positive determinative and a next determinative factor is no longer present (when a preceding determinative factor satisfies a "stop" criterion), the presence of the target nucleic acid sequence is finally determined; and when a preceding determinative factor is a positive determinative and a next determinative factor is present (when a preceding determinative factor satisfies a "go" criterion), the next determinative factor is sequentially assessed in the same manner.

<4-1> Collective determination approach

The datasets in Examples <1-1> to <1-3> were evaluated according to the collective determination approach as described above for determining the presence or absence of the target nucleic acid sequence in a sample.

In this analysis, when the three determinative factors for the three datasets were all positive determinative factors, the presence of the target nucleic acid sequence in the sample was finally determined, while when any one of the three determinative factors was a negative determinative factor, the absence of the target nucleic acid sequence in the sample was finally determined.

The results according to the collective determination approach are shown in Tables 10 and 11.

**TABLE 10**

| Case | Non-baseline subtracted curve | Slope curve | Baseline subtracted curve | Sample | |
|---|---|---|---|---|---|
| | | | | Negative | Positive |
| 1 | - | - | - | 79 | 0 |
| 2 | + | - | - | 37 | 0 |
| 3 | - | + | - | 5 | 0 |
| 4 | - | - | + | 2 | 0 |
| 5 | + | + | - | 0 | 0 |
| 8 | + | - | + | 2 | 0 |
| 7 | - | + | + | 4 | 0 |
| 8 | + | + | + | 0 | 24 |
| Total | | | | 129 | 24 |

**TABLE 11**

| Result | Multipe dataset analysis | | | | Error rate (%) | |
|---|---|---|---|---|---|---|
| | Case | Error | Normal | Total | False positive | False negative |
| Negative | Case 1~7 | 0 | 129 | 129 | 0.0% (0/153) | 0.0% (0/153) |
| Positive | Case 8 | 0 | 24 | 24 | | |

As shown in Table 10, all of 129 negative samples were non-erroneously assessed as negative samples as they were evaluated to correspond to Cases 1 to 7 in which any one of the three determinative factors is a negative determinative factor. Furthermore, all of 24 positive samples were non-erroneously assessed as positive samples as they were evaluated to correspond to Case 8 in which all of the three determinative factors are positive determinative factors. Therefore, as shown in Table 11, it was found that a false-positive error rate and a false-negative error rate for a total of 153 samples were 0.0% (0/153) and 0.0% (0/153), respectively. The results demonstrate that the multiple dataset analysis of the present invention is superior to the single dataset analysis in eliminating false positive errors.

Interestingly, it was found that all positive and negative samples were non-erroneously assessed according to the collective determination approach. The results urge us to reason that the collective determination approach using three datasets can completely eliminate the occurrence of false-positive errors.

<4-2> Sequential determination approach

In the Example, the determinative factors for the datasets in Examples <1-1> to <1-3> were assessed according to the sequential determination approach for determining the presence or the absence of the target nucleic acid sequence in the sample.

Specifically, an order was assigned to the datasets of Examples <1-1> to <1-3>, and the determinative factors obtained from the datasets were assessed sequentially in the order (the determinative factors automatically have the same order as the corresponding datasets) whether a determinative factor at a certain order satisfies a predefined "go or stop" criterion. When the determinative factor at a certain order satisfies the predefined "stop" criterion, the presence or absence of the target nucleic acid sequence was then determined, and when the determinative factor at a certain order satisfies the predefined "go"criterion, a next determinative factor was then assessed. The "stop" criterion was set as a criterion defined by which the determinative factor at a certain order is a negative factor or a criterion defined by which the determinative factor at a certain order is a positive factor and a next determinative factor is no longer present. The "go" criterion was set as a criterion defined by which the determinative factor at a certain order is a positive factor and a next determinative factor in present.

(i) Step 1: Assessment using non-baseline subtracted curve

The non-baseline subtracted curves of 24 positive samples and 129 negative samples were evaluated with predefined criteria to obtain positive or negative determinative factors. The samples having negative determinative factors were finally assessed as negative samples, and the samples having positive determinative factors were transferred to Step 2. The results are shown in Table 12.

**TABLE 12**

| | | Result | | |
| Sample | Total | Positive determinative factor | Negative determinative factor | Comment |
|---|---|---|---|---|
| Negative | 129 | 39 | 90 | 90 samples were finally assessed as negative samples |
| Positive | 24 | 24 | 0 | |

As shown in Table 12, 39 negative samples among total 129 negative samples were found to have positive determinative factors, whereas 90 negative samples were found to have negative determinative factors. All of 24 positive samples were found to have positive determinative factors. According to the present approach, 90 negative samples having negative determinative factors were finally assessed as negative samples in this step.

(ii) Step 2: Assessment using slope curve

The slope curves of 63 samples (including 39 negative samples and 24 positive samples) which had positive determinative factors in Step 1 were further evaluated with predefined criteria. The results are shown in Table 13.

**TABLE 13**

| | | Result | | |
| Sample | Total | Positive determinative factor | Negative determinative factor | Comment |
|---|---|---|---|---|
| Negative | 39 | 0 | 39 | 39 samples were finally assessed as negative samples |
| Positive | 24 | 24 | 0 | |

As shown in Table 13, 39 negative samples all were assessed to have negative determinative factors, and 24 positive samples all were assessed to have positive determinative factors. According to the present approach, all of 39 samples having negative determinative factors were assessed as negative samples in this step, and the samples having positive determinative factor were transferred to Step 3.

(iii) Step 3: Assessment using baseline subtracted curve

The baseline subtracted curves of 24 samples (including 0 negative samples and 24 positive samples) which were assessed to have positive determinative factors in Step 2 were further evaluated with predefined criteria. The results are shown in Table 14.

TABLE 14

| Sample | Total | Result | | Comment |
| | | Positive determinative factor | Negative determinative factor | |
| --- | --- | --- | --- | --- |
| Negative | 0 | 0 | 0 | |
| Positive | 24 | 24 | 0 | 24 samples were finally assessed as positive samples |

As shown in Table 14, 24 positive samples all were found to have positive determinative factors. As a next determinative factor obtained from other datasets is no longer present, 24 samples having positive determinative factors were finally assessed as positive samples in this step according to the present approach. The results above clearly showed that all positive and negative samples were non-erroneously assessed by the sequential determination approach.

The results demonstrate that the sequential determination approach using three datasets can completely eliminate any determination error. In addition, the results address that the collective and sequential determination approaches can be suitably adopted in an alternative manner.

In summary, the above-described results demonstrate that the multiple dataset analysis according to the present invention can significantly reduce any determination error compared with the single data analysis, leading to a more precise determination of the presence or absence of a target nucleic acid sequence in a sample.

In conclusion, the multiple dataset analysis according to the present invention can be effectively and reliably used to eliminate determination errors (*inter alia,* false positive errors) which are very likely to occur when using a single dataset. Furthermore, the comprehensive analysis using multiple datasets rather than only a single dataset is more effective in removing errors in various cases for determining the presence or absence of a target nucleic acid sequence. In addition to these, since the collective determination approach and the sequential determination approach exhibit substantially the same determination results, these can be alternatively adopted according to user's convenience though there are some differences in their procedures.

**Claims**

1.  A method for determining the presence or absence of a target nucleic acid sequence in a sample by a multiple dataset analysis (MDA), comprising:

    (a) performing an amplification reaction using a signal-generating means for the target nucleic acid sequence; wherein the signal-generating means comprises an oligonucleotide with a fluorescent label;
    (b) providing (i) two or more different types of datasets selected from a group consisting of: a raw dataset, a mathematically processed dataset comprising an $m^{th}$ order change amount of signal values of the raw dataset, wherein m is an integer of 1 or more, and a mathematically processed dataset comprising signal values subtracted by a baseline determined to remove background signal values of the raw dataset, said two or more different types of datasets representing an outcome of the amplification reaction using the signal-generating means for the target nucleic acid sequence; wherein each of the two or more different types of datasets comprises a plurality of data points, each data point having a pair of coordinate values comprising a cycle number and a signal value at the cycle number, and (ii) two or more determinative factors, wherein each of the determinative factors is a positive or negative determinative factor obtained by employing as a predefined criterion a threshold for differentiating a positive sample or a negative sample to each of the two or more different types of datasets; wherein the positive determinative factor represents the probability of the presence of the target nucleic acid sequence and the negative determinative factor represents the probability of the absence of the target nucleic acid sequence; and
    (c) determining the presence or absence of the target nucleic acid sequence in the sample by using the two or more determinative factors; wherein if all of the two or more determinative factors are positive determinative factors, the presence of the target nucleic acid sequence in the sample is determined, and if not, the absence of the target nucleic acid sequence in the sample is determined.

2. The method of claim 1, wherein the amplification reaction in step (a) is a polymerase chain reaction (PCR) or a real-time PCR.

3. The method of claim 1, wherein the determination of the presence or absence of the target nucleic acid sequence is performed by assigning an order to the datasets, and assessing the determinative factors having the same order as the corresponding datasets sequentially in the order with a predefined "go or stop" criterion.

4. The method of claim 3, wherein the "go or stop" criterion is defined by whether a determinative factor is positive or negative and whether a next determinative factor is present or not.

5. The method of claim 3, wherein the "go or stop" criterion comprises (i) a "stop" criterion defined by which a determinative factor is negative, (ii) a "stop" criterion defined by which a determinative factor is positive and a next determinative factor is no longer present, and (iii) a "go" criterion defined by which a determinative factor is positive and a next determinative factor is present; wherein when the determinative factor at a certain order satisfies the "stop" criterion defined by (i), the absence of the target nucleic acid sequence is determined; when it satisfies the "stop" criterion defined by (ii), the presence of the target nucleic acid sequence is determined; and when it satisfies the "go" criterion defined by (iii), a next determinative factor is then assessed, or wherein the "go or stop" criterion comprises (i) a "stop" criterion defined by which a determinative factor is positive, (ii) a "stop" criterion defined by which a determinative factor is negative and a next determinative factor is no longer present, and (iii) a "go" criterion defined by which a determinative factor is negative and a next determinative factor is present; wherein when the determinative factor at a certain order satisfies the "stop" criterion defined by (i), the presence of the target nucleic acid sequence is determined; when it satisfies the "stop" criterion defined by (ii), the absence of the target nucleic acid sequence is determined; and when it satisfies the "go" criterion defined by (iii), a next determinative factor is then assessed.

6. A computer readable storage medium containing instructions to configure a processor to perform a method for determining the presence or absence of a target nucleic acid sequence in a sample by a multiple dataset analysis (MDA), the method comprising:

   (a) receiving an outcome of an amplification reaction using a signal-generating means for the target nucleic acid sequence;
   (b) providing (i) two or more different types of datasets selected from a group consisting of: a raw dataset, a mathematically processed dataset comprising an $m^{th}$ order change amount of signal values of the raw dataset, wherein m is an integer of 1 or more, and a mathematically processed dataset comprising signal values subtracted by a baseline determined to remove background signal values of the raw dataset, said two or more different types of datasets representing an outcome of the amplification reaction using the signal-generating means for the target nucleic acid sequence; wherein each of the two or more different types of datasets comprises a plurality of data points, each data point having a pair of coordinate values comprising a cycle number and a signal value at the cycle number, and (ii) two or more determinative factors, wherein the determinative factors are obtained by employing as a predefined criterion a threshold for differentiating a positive sample or a negative sample to each of the two or more different types of datasets; two or more determinative factors, wherein each of the determinative factors is a positive or negative determinative factor obtained by employing as a predefined criterion a threshold for differentiating a positive sample or a negative sample to each of the two or more different types of datasets; wherein the positive determinative factor represents the probability of the presence of the target nucleic acid sequence and the negative determinative factor represents the probability of the absence of the target nucleic acid sequence; and
   (c) determining the presence or absence of the target nucleic acid sequence in the sample by using the two or more determinative factors; wherein if all of the two or more determinative factors are positive determinative factors, the presence of the target nucleic acid sequence in the sample is determined, and if not, the absence of the target nucleic acid sequence in the sample is determined.

7. A device for determining the presence or absence of a target nucleic acid sequence in a sample by a multiple dataset analysis (MDA), the device comprising:

   (a) a computer processor; and (b) the computer readable storage medium according to claim 6 which is coupled to said computer processor.

8. A computer program to be stored in a computer readable storage medium containing instructions to configure a processor to perform a method for determining the presence or absence of a target nucleic acid sequence in a sample by a multiple dataset analysis (MDA), the method comprising:

(a) receiving an outcome of an amplification reaction using a signal-generating means for the target nucleic acid sequence;
(b) providing (i) two or more different types of datasets selected from a group consisting of: a raw dataset, a mathematically processed dataset comprising an m$^{th}$ order change amount of signal values of the raw dataset, wherein m is an integer of 1 or more, and a mathematically processed dataset comprising signal values subtracted by a baseline determined to remove background signal values of the raw dataset, said two or more different types of datasets representing an outcome of the amplification reaction using the signal-generating means for the target nucleic acid sequence; wherein each of the two or more different types of datasets comprises a plurality of data points, each data point having a pair of coordinate values comprising a cycle number and a signal value at the cycle number, and (ii) two or more determinative factors, wherein each of the determinative factors is a positive or negative determinative factor obtained by employing as a predefined criterion a threshold for differentiating a positive sample or a negative sample to each of the two or more different types of datasets; wherein the positive determinative factor represents the probability of the presence of the target nucleic acid sequence and the negative determinative factor represents the probability of the absence of the target nucleic acid sequence; and
(c) determining the presence or absence of the target nucleic acid sequence in the sample by using the two or more determinative factors; wherein if all of the two or more determinative factors are positive determinative factors, the presence of the target nucleic acid sequence in the sample is determined, and if not, the absence of the target nucleic acid sequence in the sample is determined.

**Patentansprüche**

1. Verfahren zum Bestimmen der Anwesenheit oder Abwesenheit einer Zielnukleinsäuresequenz in einer Probe durch eine Mehrfachdatensatzanalyse (MDA), umfassend:

(a) Durchführen einer Amplifikationsreaktion unter Verwendung eines Signalerzeugungsmittels für die Zielnukleinsäuresequenz; wobei das Signalerzeugungsmittel ein Oligonukleotid mit einer fluoreszierenden Markierung umfasst;
(b) Bereitstellen (i) von zwei oder mehr verschiedenen Arten von Datensätzen, ausgewählt aus einer Gruppe, bestehend aus: einem Rohdatensatz, einem mathematisch verarbeiteten Datensatz, der eine Änderungsmenge m-ter Ordnung von Signalwerten des Rohdatensatzes umfasst, wobei m eine ganze Zahl von 1 oder mehr ist, und einem mathematisch verarbeiteten Datensatz, der Signalwerte umfasst, die von einer Grundlinie subtrahiert werden, die bestimmt wird, um Hintergrundsignalwerte des Rohdatensatzes zu entfernen, wobei die zwei oder mehr verschiedenen Arten von Datensätzen ein Ergebnis der Amplifikationsreaktion unter Verwendung des Signalerzeugungsmittels für die Zielnukleinsäuresequenz darstellen; wobei jeder der zwei oder mehr verschiedenen Arten von Datensätzen eine Vielzahl von Datenpunkten umfasst, wobei jeder Datenpunkt ein Paar von Koordinatenwerten aufweist, das eine Zykluszahl und einen Signalwert bei der Zykluszahl umfasst, und (ii) zwei oder mehr determinative Faktoren, wobei jeder der determinativen Faktoren ein positiver oder negativer determinativer Faktor ist, der erhalten wird, indem als ein vordefiniertes Kriterium ein Schwellenwert zum Differenzieren einer positiven Probe oder einer negativen Probe zu jedem der zwei oder mehr verschiedenen Arten von Datensätzen verwendet wird; wobei der positive determinative Faktor die Wahrscheinlichkeit der Anwesenheit der Zielnukleinsäuresequenz darstellt und der negative determinative Faktor die Wahrscheinlichkeit der Abwesenheit der Zielnukleinsäuresequenz darstellt; und
(c) Bestimmen der Anwesenheit oder Abwesenheit der Zielnukleinsäuresequenz in der Probe unter Verwendung der zwei oder mehr determinativen Faktoren; wobei, wenn alle der zwei oder mehr determinativen Faktoren positive determinative Faktoren sind, die Anwesenheit der Zielnukleinsäuresequenz in der Probe bestimmt wird, und wenn nicht, die Abwesenheit der Zielnukleinsäuresequenz in der Probe bestimmt wird.

2. Verfahren nach Anspruch 1, wobei die Amplifikationsreaktion in Schritt (a) eine Polymerasekettenreaktion (PCR) oder eine Echtzeit-PCR ist.

3. Verfahren nach Anspruch 1, wobei die Bestimmung der Anwesenheit oder Abwesenheit der Zielnukleinsäuresequenz durch Zuordnen einer Reihenfolge zu den Datensätzen und Bewerten der determinativen Faktoren mit der gleichen Reihenfolge wie die entsprechenden Datensätze sequentiell in der Reihenfolge mit einem vordefinierten "Go-or-Stop"-Kriterium durchgeführt wird.

4. Verfahren nach Anspruch 3, wobei das "Go-or-Stop"-Kriterium dadurch definiert ist, ob ein determinativer Faktor positiv oder negativ ist und ob ein nächster determinativer Faktor vorhanden ist oder nicht.

5. Verfahren nach Anspruch 3, wobei das "Go-or-Stop"-Kriterium (i) ein "Stop"-Kriterium, das dadurch definiert ist, dass ein determinativer Faktor negativ ist, (ii) ein "Stop"-Kriterium, das dadurch definiert ist, dass ein determinativer Faktor positiv ist und ein nächster determinativer Faktor nicht mehr vorhanden ist, und (iii) ein "Go"-Kriterium, das dadurch definiert ist, dass ein determinativer Faktor positiv ist und ein nächster determinativer Faktor vorhanden ist, umfasst; wobei, wenn der determinative Faktor in einer bestimmten Reihenfolge das "Stop"-Kriterium, das durch (i) definiert ist, erfüllt, die Abwesenheit der Zielnukleinsäuresequenz bestimmt wird; wenn er das "Stop"-Kriterium, das durch (ii) definiert ist, erfüllt, die Anwesenheit der Zielnukleinsäuresequenz bestimmt wird; und wenn er das "Go"-Kriterium, das durch (iii) definiert ist, erfüllt, dann ein nächster determinativer Faktor bewertet wird, oder wobei das "Go-or-Stop"-Kriterium (i) ein "Stop"-Kriterium, das dadurch definiert ist, dass ein determinativer Faktor positiv ist, (ii) ein "Stop"-Kriterium, das dadurch definiert ist, dass ein determinativer Faktor negativ ist und ein nächster determinativer Faktor nicht mehr vorhanden ist, und (iii) ein "Go"-Kriterium, das dadurch definiert ist, dass ein determinativer Faktor negativ ist und ein nächster determinativer Faktor vorhanden ist, umfasst; wobei, wenn der determinative Faktor in einer bestimmten Reihenfolge das "Stop"-Kriterium, das durch (i) definiert ist, erfüllt, die Anwesenheit der Zielnukleinsäuresequenz bestimmt wird; wenn er das "Stop"-Kriterium, das durch (ii) definiert ist, erfüllt, die Abwesenheit der Zielnukleinsäuresequenz bestimmt wird; und wenn er das "Go"-Kriterium, das durch (iii) definiert ist, erfüllt, dann ein nächster determinativer Faktor bewertet wird.

6. Computerlesbares Speichermedium, das Anweisungen enthält, um einen Prozessor zu konfigurieren, um ein Verfahren zum Bestimmen der Anwesenheit oder Abwesenheit einer Zielnukleinsäuresequenz in einer Probe durch eine Mehrfachdatensatzanalyse (MDA) durchzuführen, wobei das Verfahren umfasst:

(a) Empfangen eines Ergebnisses einer Amplifikationsreaktion unter Verwendung eines Signalerzeugungsmittels für die Zielnukleinsäuresequenz;
(b) Bereitstellen (i) von zwei oder mehr verschiedenen Arten von Datensätzen, ausgewählt aus einer Gruppe, bestehend aus: einem Rohdatensatz, einem mathematisch verarbeiteten Datensatz, der eine Änderungsmenge m-ter Ordnung von Signalwerten des Rohdatensatzes umfasst, wobei m eine ganze Zahl von 1 oder mehr ist, und einem mathematisch verarbeiteten Datensatz, der Signalwerte umfasst, die von einer Grundlinie subtrahiert werden, die bestimmt wird, um Hintergrundsignalwerte des Rohdatensatzes zu entfernen, wobei die zwei oder mehr verschiedenen Arten von Datensätzen ein Ergebnis der Amplifikationsreaktion unter Verwendung des Signalerzeugungsmittels für die Zielnukleinsäuresequenz darstellen; wobei jeder der zwei oder mehr verschiedenen Arten von Datensätzen eine Vielzahl von Datenpunkten umfasst, wobei jeder Datenpunkt ein Paar von Koordinatenwerten aufweist, das eine Zykluszahl und einen Signalwert bei der Zykluszahl umfasst, und (ii) zwei oder mehr determinative Faktoren, wobei die determinativen Faktoren erhalten werden, indem als ein vordefiniertes Kriterium ein Schwellenwert zum Differenzieren einer positiven Probe oder einer negativen Probe zu jedem der zwei oder mehr verschiedenen Arten von Datensätzen verwendet wird; zwei oder mehr determinative Faktoren, wobei jeder der determinativen Faktoren ein positiver oder negativer determinativer Faktor ist, der erhalten wird, indem als ein vordefiniertes Kriterium ein Schwellenwert zum Differenzieren einer positiven Probe oder einer negativen Probe zu jedem der zwei oder mehr verschiedenen Arten von Datensätzen verwendet wird; wobei der positive determinative Faktor die Wahrscheinlichkeit der Anwesenheit der Zielnukleinsäuresequenz darstellt und der negative determinative Faktor die Wahrscheinlichkeit der Abwesenheit der Zielnukleinsäuresequenz darstellt; und
(c) Bestimmen der Anwesenheit oder Abwesenheit der Zielnukleinsäuresequenz in der Probe unter Verwendung der zwei oder mehr determinativen Faktoren; wobei, wenn alle der zwei oder mehr determinativen Faktoren positive determinative Faktoren sind, die Anwesenheit der Zielnukleinsäuresequenz in der Probe bestimmt wird, und wenn nicht, die Abwesenheit der Zielnukleinsäuresequenz in der Probe bestimmt wird.

7. Vorrichtung zum Bestimmen der Anwesenheit oder Abwesenheit einer Zielnukleinsäuresequenz in einer Probe durch eine Mehrfachdatensatzanalyse (MDA), wobei die Vorrichtung umfasst:

(a) einen Computerprozessor; und (b) das computerlesbare Speichermedium nach Anspruch 6, das mit dem Computerprozessor gekoppelt ist.

8. Computerprogramm, das in einem computerlesbaren Speichermedium gespeichert werden soll, das Anweisungen enthält, um einen Prozessor zu konfigurieren, um ein Verfahren zum Bestimmen der Anwesenheit oder Abwesenheit einer Zielnukleinsäuresequenz in einer Probe durch eine Mehrfachdatensatzanalyse (MDA) durchzuführen, wobei das Verfahren umfasst:

(a) Empfangen eines Ergebnisses einer Amplifikationsreaktion unter Verwendung eines Signalerzeugungsmittels für die Zielnukleinsäuresequenz;

(b) Bereitstellen (i) von zwei oder mehr verschiedenen Arten von Datensätzen, ausgewählt aus einer Gruppe, bestehend aus: einem Rohdatensatz, einem mathematisch verarbeiteten Datensatz, der eine Änderungsmenge m-ter Ordnung von Signalwerten des Rohdatensatzes umfasst, wobei m eine ganze Zahl von 1 oder mehr ist, und einem mathematisch verarbeiteten Datensatz, der Signalwerte umfasst, die von einer Grundlinie subtrahiert werden, die bestimmt wird, um Hintergrundsignalwerte des Rohdatensatzes zu entfernen, wobei die zwei oder mehr verschiedenen Arten von Datensätzen ein Ergebnis der Amplifikationsreaktion unter Verwendung des Signalerzeugungsmittels für die Zielnukleinsäuresequenz darstellen; wobei jeder der zwei oder mehr verschiedenen Arten von Datensätzen eine Vielzahl von Datenpunkten umfasst, wobei jeder Datenpunkt ein Paar von Koordinatenwerten aufweist, das eine Zykluszahl und einen Signalwert bei der Zykluszahl umfasst, und (ii) zwei oder mehr determinative Faktoren, wobei jeder der determinativen Faktoren ein positiver oder negativer determinativer Faktor ist, der erhalten wird, indem als ein vordefiniertes Kriterium ein Schwellenwert zum Differenzieren einer positiven Probe oder einer negativen Probe zu jedem der zwei oder mehr verschiedenen Arten von Datensätzen verwendet wird; wobei der positive determinative Faktor die Wahrscheinlichkeit der Anwesenheit der Zielnukleinsäuresequenz darstellt und der negative determinative Faktor die Wahrscheinlichkeit der Abwesenheit der Zielnukleinsäuresequenz darstellt; und

(c) Bestimmen der Anwesenheit oder Abwesenheit der Zielnukleinsäuresequenz in der Probe unter Verwendung der zwei oder mehr determinativen Faktoren; wobei, wenn alle der zwei oder mehr determinativen Faktoren positive determinative Faktoren sind, die Anwesenheit der Zielnukleinsäuresequenz in der Probe bestimmt wird, und wenn nicht, die Abwesenheit der Zielnukleinsäuresequenz in der Probe bestimmt wird.

**Revendications**

1. Procédé pour déterminer la présence ou l'absence d'une séquence d'acide nucléique cible dans un échantillon par le biais d'une analyse d'ensembles de données multiples (MDA), comprenant le fait de :

(a) effectuer une réaction d'amplification à l'aide d'un moyen de génération de signal pour la séquence d'acide nucléique cible ; dans lequel le moyen de génération de signal comprend un oligonucléotide avec un marqueur fluorescent ;

(b) fournir (i) deux types différents d'ensembles de données ou plus sélectionnés parmi un groupe consistant en : un ensemble de données brute, un ensemble de données traité mathématiquement comprenant une quantité de variation d'ordre m de valeurs de signal de l'ensemble de données brute, où m est un entier égal ou supérieur à 1, et un ensemble de données traité mathématiquement comprenant des valeurs de signal soustraites d'une ligne de base déterminée pour supprimer des valeurs de signal d'arrière-plan de l'ensemble de données brute, lesdits deux types différents d'ensembles de données ou plus représentant un résultat de la réaction d'amplification utilisant le moyen de génération de signal pour la séquence d'acide nucléique cible ; dans lequel chacun des deux types différents d'ensembles de données ou plus comprend une pluralité de points de données, chaque point de données ayant une paire de valeurs de coordonnées comprenant un numéro de cycle et une valeur de signal au niveau du numéro de cycle, et (ii) deux facteurs déterminants ou plus, dans lequel chacun des facteurs déterminants est un facteur déterminant positif ou négatif obtenu en utilisant, comme critère prédéfini, un seuil pour différencier un échantillon positif ou un échantillon négatif pour chacun des deux types différents d'ensembles de données ou plus ; dans lequel le facteur déterminant positif représente la probabilité de la présence de la séquence d'acide nucléique cible et le facteur déterminant négatif représente la probabilité de l'absence de la séquence d'acide nucléique cible ; et

(c) déterminer la présence ou l'absence de la séquence d'acide nucléique cible dans l'échantillon en utilisant les deux facteurs déterminants ou plus ; dans lequel, si tous les deux facteurs déterminants ou plus sont des facteurs déterminants positifs, la présence de la séquence d'acide nucléique cible dans l'échantillon est déterminée, et sinon, l'absence de la séquence d'acide nucléique cible dans l'échantillon est déterminée.

2. Procédé selon la revendication 1, dans lequel la réaction d'amplification à l'étape (a) est une réaction en chaîne par polymérase (PCR) ou une PCR en temps réel.

**3.** Procédé selon la revendication 1, dans lequel la détermination de la présence ou de l'absence de la séquence d'acide nucléique cible est effectuée en attribuant un ordre aux ensembles de données et en évaluant les facteurs déterminants ayant le même ordre que les ensembles de données correspondants, de manière séquentielle, dans l'ordre, avec un critère « poursuite ou arrêt » prédéfini.

**4.** Procédé selon la revendication 3, dans lequel le critère « poursuite ou arrêt » est défini par le fait qu'un facteur déterminant est positif ou négatif et qu'un facteur déterminant suivant est présent ou non.

**5.** Procédé selon la revendication 3, dans lequel le critère « poursuite ou arrêt » comprend (i) un critère « arrêt » défini par lequel un facteur déterminant est négatif, (ii) un critère « arrêt » défini par le fait qu'un facteur déterminant est positif et qu'un facteur déterminant suivant n'est plus présent, et (iii) un critère « poursuite » défini par le fait qu'un facteur déterminant est positif et qu'un facteur déterminant suivant est présent ; dans lequel, lorsque le facteur déterminant à un ordre donné satisfait le critère « arrêt » défini par (i), l'absence de la séquence d'acide nucléique cible est déterminée ; lorsqu'il satisfait le critère « arrêt » défini par (ii), la présence de la séquence d'acide nucléique cible est déterminée ; et lorsqu'il satisfait au critère « poursuite » défini par (iii), un facteur déterminant suivant est alors évalué, ou dans lequel le critère « poursuite ou arrêt » comprend (i) un critère « arrêt » défini par lequel un facteur déterminant est positif, (ii) un critère « arrêt » défini par lequel un facteur déterminant est négatif et un facteur déterminant suivant n'est plus présent, et (iii) un critère « poursuite » défini par lequel un facteur déterminant est négatif et un facteur déterminant suivant est présent ; dans lequel lorsque le facteur déterminant à un ordre donné satisfait le critère « arrêt » défini par (i), la présence de la séquence d'acide nucléique cible est déterminée ; lorsqu'il satisfait le critère « arrêt » défini par (ii), l'absence de la séquence d'acide nucléique cible est déterminée ; et lorsqu'il satisfait le critère « poursuite » défini par (iii), un facteur déterminant suivant est alors évalué.

**6.** Support de stockage lisible par ordinateur contenant des instructions pour configurer un processeur afin de mettre en œuvre un procédé pour déterminer la présence ou l'absence d'une séquence d'acide nucléique cible dans un échantillon par le biais d'une analyse d'ensembles de données multiples (MDA), le procédé comprenant le fait de :

(a) recevoir un résultat d'une réaction d'amplification utilisant un moyen de génération de signal pour la séquence d'acide nucléique cible ;
(b) fournir (i) deux types différents d'ensembles de données ou plus sélectionnés parmi un groupe consistant en : un ensemble de données brute, un ensemble de données traité mathématiquement comprenant une quantité de variation d'ordre m de valeurs de signal de l'ensemble de données brute, où m est un entier égal ou supérieur à 1, et un ensemble de données traité mathématiquement comprenant des valeurs de signal soustraites d'une ligne de base déterminée pour supprimer des valeurs de signal d'arrière-plan de l'ensemble de données brute, lesdits deux types différents d'ensembles de données ou plus représentant un résultat de la réaction d'amplification utilisant le moyen de génération de signal pour la séquence d'acide nucléique cible ; dans lequel chacun des deux types différents d'ensembles de données ou plus comprend une pluralité de points de données, chaque point de données ayant une paire de valeurs de coordonnées comprenant un numéro de cycle et une valeur de signal au niveau du numéro de cycle, et (ii) deux facteurs déterminants ou plus, dans lequel les facteurs déterminants sont obtenus en utilisant, comme critère prédéfini, un seuil pour différencier un échantillon positif ou un échantillon négatif pour chacun des deux types différents d'ensembles de données ou plus ; deux facteurs déterminants ou plus, dans lesquels chacun des facteurs déterminants est un facteur déterminant positif ou négatif obtenu en utilisant, comme critère prédéfini, un seuil pour différencier un échantillon positif ou un échantillon négatif pour chacun des deux types différents d'ensembles de données ou plus ; dans lesquels le facteur déterminant positif représente la probabilité de la présence de la séquence d'acide nucléique cible et le facteur déterminant négatif représente la probabilité de l'absence de la séquence d'acide nucléique cible ; et
(c) déterminer la présence ou l'absence de la séquence d'acide nucléique cible dans l'échantillon en utilisant les deux facteurs déterminants ou plus ; dans lequel, si tous les deux facteurs déterminants ou plus sont des facteurs déterminants positifs, la présence de la séquence d'acide nucléique cible dans l'échantillon est déterminée, et sinon, l'absence de la séquence d'acide nucléique cible dans l'échantillon est déterminée.

**7.** Dispositif permettant de déterminer la présence ou l'absence d'une séquence d'acide nucléique cible dans un échantillon par le biais d'une analyse d'ensembles de données multiples (MDA), le dispositif comprenant :

(a) un processeur informatique ; et (b) le support de stockage lisible par ordinateur selon la revendication 6, qui est couplé audit processeur informatique.

**8.** Programme informatique destiné à être stocké sur un support de stockage lisible par ordinateur contenant des instructions pour configurer un processeur afin de mettre en œuvre un procédé pour déterminer la présence ou l'absence d'une séquence d'acide nucléique cible dans un échantillon par une analyse d'ensembles de données multiples (MDA), le procédé comprenant le fait de :

(a) recevoir un résultat d'une réaction d'amplification utilisant un moyen de génération de signal pour la séquence d'acide nucléique cible ;

(b) fournir (i) deux types différents d'ensembles de données ou plus sélectionnés parmi un groupe consistant en : un ensemble de données brute, un ensemble de données traité mathématiquement comprenant une quantité de variation d'ordre m de valeurs de signal de l'ensemble de données brute, où m est un entier égal ou supérieur à 1, et un ensemble de données traité mathématiquement comprenant des valeurs de signal soustraites d'une ligne de base déterminée pour supprimer des valeurs de signal d'arrière-plan de l'ensemble de données brute, lesdits deux types différents d'ensembles de données ou plus représentant un résultat de la réaction d'amplification utilisant le moyen de génération de signal pour la séquence d'acide nucléique cible ; dans lequel chacun des deux types différents d'ensembles de données ou plus comprend une pluralité de points de données, chaque point de données ayant une paire de valeurs de coordonnées comprenant un numéro de cycle et une valeur de signal au niveau du numéro de cycle, et (ii) deux facteurs déterminants ou plus, dans lequel chacun des facteurs déterminants est un facteur déterminant positif ou négatif obtenu en utilisant, comme critère prédéfini, un seuil pour différencier un échantillon positif ou un échantillon négatif pour chacun des deux types différents d'ensembles de données ou plus ; dans lequel le facteur déterminant positif représente la probabilité de la présence de la séquence d'acide nucléique cible et le facteur déterminant négatif représente la probabilité de l'absence de la séquence d'acide nucléique cible ; et

(c) déterminer la présence ou l'absence de la séquence d'acide nucléique cible dans l'échantillon en utilisant les deux facteurs déterminants ou plus ; dans lequel, si tous les deux facteurs déterminants ou plus sont des facteurs déterminants positifs, la présence de la séquence d'acide nucléique cible dans l'échantillon est déterminée, et sinon, l'absence de la séquence d'acide nucléique cible dans l'échantillon est déterminée.

# Fig. 1

## *100*

*200* — 
```
Perform an amplification reaction
```

*300* — 
```
Provide (i) a dataset pool; and
(ii) two or more determinative factors
```

*400* — 
```
Determine the presence or absence of
a target analyte in a sample by using
at least two of the determinative factors
```

# Fig. 2

*100*

| Amplification reaction | *200* |

| 1st dataset | 2nd dataset | 3rd dataset | |
| 1st determinative factor | 2nd determinative factor | 3rd determinative factor | *300* |

| 1st | 2nd | 3rd | Collective Determination | |
|-----|-----|-----|--------------------------|---|
| + | + | + | presence | |
| - | + | + | absence | *400* |
| + | - | + | absence | |
| + | + | - | absence | |
| - | - | + | absence | |
| - | + | - | absence | |
| + | - | - | absence | |
| - | - | - | absence | |

# Fig. 3

```
┌─────────────────────────────┐
│  1ˢᵗ determinative factor   │
│         (positive)          │
└─────────────────────────────┘
              │
              ▼
        ╱─────────────╲
       ╱  "go or stop"  ╲
       ╲   criterion    ╱
        ╲─────────────╱
              │ "go"
              ▼
┌─────────────────────────────┐
│  2ⁿᵈ determinative factor   │
│         (positive)          │
└─────────────────────────────┘
              │
              ▼
        ╱─────────────╲
       ╱  "go or stop"  ╲
       ╲   criterion    ╱
        ╲─────────────╱
              │ "go"
              ▼
┌─────────────────────────────┐
│  3ʳᵈ determinative factor   │
│         (positive)          │
└─────────────────────────────┘
              │
              ▼
        ╱─────────────╲
       ╱  "go or stop"  ╲───── "stop" ──→ presence
       ╲   criterion    ╱
        ╲─────────────╱
```

# Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8906700 A, Miller, H. I.
- WO 8810315 A, Gingeras T.R.
- US 4683195 A, Mullis
- US 4683202 A
- US 4800159 A
- US 7720611 B
- EP 2653558 A
- EP 1798650 A
- US 8560247 B
- US 20150186598 A
- US 5210015 A
- US 6117635 A
- US 7537886 B
- US 6977295 B
- US 7348141 B
- US 5876930 A
- WO 2012096523 A
- WO 2013115442 A
- WO 2014104818 A
- WO 2011037306 A
- US 6358691 B
- US 6194149 B

### Non-patent literature cited in the description

- **KWOH, D. et al.** *Proc. Natl. Acad. Sci. U.S.A.*, 1989, vol. 86, 1173
- **SAIKI et al.** *Science*, 1985, vol. 230, 1350-1354
- **ALEXEY LARIONOVET.** *BMC Bioinformatics*, 2005, vol. 6, 62
- **SAMBROOK, J. et al.** Molecular Cloning. A Laboratory Manual. Cold Spring Harbor Press, 2001
- **TYAGIET.** *Nature Biotechnology*
- **WHITCOMBE et al.** *Nature Biotechnology*, 1999, vol. 17, 804-807
- **NAZARENKOET.** *Nucleic Acids Research*, 1997, vol. 25 (12), 2516-2521
- **DUCK P et al.** *Biotechniques*, 1990, vol. 9, 142-148
- **SHERRILL CB et al.** *Journal of the American Chemical Society*, 2004, vol. 126, 4550-4556
- **D. J. FRENCH et al.** *Molecular and Cellular Probes*, 2001, vol. 13, 363-374
- **BERNARD PS et al.** *ClinChem*, 2000, vol. 46, 147-148
- *PCR Protocols: A Guide to Methods and Applications*, 1990
- **WALKER et al.** *Nucleic Acids Res.*, 1992, vol. 20 (7), 1691-6
- *Walker PCR Methods Appl*, 1993, vol. 3 (1), 1-6
- **PHYFFER et al.** *J. Clin. Microbiol.*, 1996, vol. 34, 834-841
- **VUORINEN et al.** *J. Clin. Microbiol.*, 1995, vol. 33, 1856-1859
- **COMPTON.** *Nature*, 1991, vol. 350 (6313), 91-2
- **LISBY.** *Mol. Biotechnol.*, 1999, vol. 12 (1), 75-99
- **HATCH et al.** *Genet. Anal.*, 1999, vol. 15 (2), 35-40
- **LIZARDI et al.** *BiolTechnology*, 1988, vol. 6, 1197